# EUROPEAN PATENT APPLICATION

(11) **EP 2 383 341 A1**
(43) Date of publication of application: **02.11.2011**
(21) Application number: 11171243.6
(22) Date of filing: 12.06.2007
(51) Int. Cl.: C12N 15/113, A61K 31/713, A61P 27/00

(54) **Compositions and methods for siRNA inhibition of angiogenesis**

(30) Priority: 12.06.2006 US 804500; 08.09.2006 US 824972; 06.11.2006 US 864440
(62) Divisional of application: 07812121.7
(71) Applicant: Exegenics, Inc., D/b/a Opko Health, Inc., Philadelphia, Pennsylvania 19104 (US)
(72) Inventor: Reich, Samuel Jotham, Miami Beach, FL 33140 (US)
(74) Representative: Ouzman, Beverley Nicola Claire

(57) **Abstract**

Embodiments of methods of using siRNAs targeting VEGF including an siRNA defined by SEQ ID NO: 77 and SEQ ID NO: 78 to stablize visual acuity in a subject, to inhibit choroidal neovascularization lesions, to treat age-related ;macular degeneration, to treat diabetic macular edema, and to decrease foveal thickness in subjects are disclosed. Additionally, methods of treating age-related macular degeneration and diabetic macular edema by administering combinatorial therapy comprising an siRNA targeting VEGF and a non-siRNA VEGF antagonist are described.

SEQUENCE LISTING

<110> Exegenics, Inc., D/b/a Opko Health, Inc

<120> Compositions and Methods for siRNA Inhibition of Angiogenesis

<130> P120943.EP.01

<140> Not yet assigned
<141> 2007-06-12

<150> US 60/804,500
<151> 2006-06-12

<150> US 60/824,972
<151> 2006-09-08

<150> US 60/864,440
<151> 2006-11-06

<160> 85

<170> PatentIn version 3.3

<210> 1
<211> 2250
<212> DNA
<213> Mus musculus

<400> 1
tgagccaggc tggcaggaag gagcctccct cagggtttcg ggaaccagac ctctcaccgg 60
aaagaccgat taaccatgtc accaccacgc catcatcgtc accgttgaca gaacagtcct 120
taatccagaa agcctgacat gaaggaagag gagactcttc gaggagcact ttgggtccgg 180
agggcgagac tccggcagac gcattcccgg gcaggtgacc aagcacggtc cctcgtggga 240
ctggattcgc cattttctta tatctgctgc taaatcgcca agcccggaag attagggttg 300
tttctgggat tcctgtagac acacccaccc acatacacac atatatatat attatatata 360
taaataaata tatatgtttt atatataaaa tatatatata ttcttttttt taaattaact 420
ctgctaatgt tattggtgtc ttcactggat atgtttgact gctgtggact tgtgttggga 480
ggaggatgtc ctcactcgga tgccgacatg ggagacaatg ggatgaaagg cttcagtgtg 540
gtctgagaga ggccgaagtc cttttgcctg ccggggagca agcaaggcca gggcacgggg 600
gcacattggc tcacttccag aaacacgaca aacccattcc tggccctgag tcaagaggac 660
agagagacag atgatgacac agaaagagat aaagatgccg gttccaacca gaagtttggg 720
gagcctcagg acatggcatg ctttgtggat ccccatgata gtctacaaaa gcaccccgcc 780
cctctgggca ctgcctggaa gaatcgggag cctggccagc cttcagctcg ctcctccact 840
tctgaggggc ctaggaggcc tcccacaggt gtcccggcaa gagaagacac ggtggtggaa 900
gaagaggcct ggtaatggcc cctcctcctg ggaccccttc gtcctctcct taccccacct 960
cctgggtaca gcccaggagg accttgtgtg atcagaccat tgaaaccact aattctgtcc 1020
ccaggagact tggctctgtg tgtgagtggc ttacccttcc tcatcttccc ttcccaaggc 1080
acagagcaat ggggcaggac ccgcaagccc ctcacggagg cagagaaaag agaaagtgtt 1140
ttatatacgg tacttattta atagcccttt ttaattagaa attaaaacag ttaatttaat 1200
taaagagtag ggtttttttc agtattcttg gttaatattt aatttcaact atttatgaga 1260
tgtatctctc gctctctctt atttgtactt atgtgtgtgt gtgtgtgtgt gtgtgtgtgt 1320
gtgtgtgtgt gtatgaaatc tgtgtttcca atctctctct cccagatcgg tgacagtcac 1380
tagcttgtcc tgagaagata tttaattttg ctaacactca gctctgccct cccttgtccc 1440
caccacacat tcctttgaaa taaggtttca atatacattt acatactata tatatatttg 1500
gcaacttgtg tttgtatata aatatatata tatatatatg tttatgtata tatgtgattc 1560
tgataaaata gacattgcta ttctgttttt tatatgtaaa aacaaaacaa gaaaaataga 1620
gaattctaca tactaaatct ctctcctttt ttaattttaa tatttgttat catttattta 1680
ttggtgctac tgtttatccg taataattgt gggggaaaaa gatattaaca tcacgtcttt 1740
gtctctagag cagttttccg agatattccg tagtacatat ttatttttaa acagcaacaa 1800
agaaatacag atatatctta aaaaaaaagc attttgtatt aaagaattga attctgatct 1860
caaagctctc cctggtctct ccttctctcc tgggccctcc tgtctcgctt tccctcctcc 1920
tttggggtac atagtttttg tcttaggttt gagaagcagt ccctggagta gaatatgggg 1980
tgacccatcc attcctgggc ggaggggaga tggctccttt gccaagggtc ctcacactac 2040
gtggtactct gttccttgtc agacaaggat gggggcatgt ctccaggtgc taactggaga 2100
tcggagagag ctgttggctg cagctggcca ggatttgggc atgccgggga catgggaggc 2160

tgtgagccca gcatgcagtt tacttctggg tgctaaatgg aagagtccag taaaaagagt 2220
cttgcccatg ggattccatt ccgctttgtg 2250

<210> 2
<211> 444
<212> DNA
<213> Homo sapiens

<400> 2
atgaactttc tgctgtcttg ggtgcattgg agccttgcct tgctgctcta cctccaccat 60
gccaagtggt cccaggctgc acccatggca gaaggaggag ggcagaatca tcacgaagtg 120
gtgaagttca tggatgtcta tcagcgcagc tactgccatc caatcgagac cctggtggac 180
atcttccagg agtaccctga tgagatcgag tacatcttca agccatcctg tgtgcccctg 240
atgcgatgcg ggggctgctg caatgacgag ggcctggagt gtgtgcccac tgaggagtcc 300
aacatcacca tgcagattat gcggatcaaa cctcaccaag gccagcacat aggagagatg 360
agcttcctac agcacaacaa atgtgaatgc agaccaaaga aagatagagc aagacaagaa 420
aaatgtgaca agccgaggcg gtga 444

<210> 3
<211> 576
<212> DNA
<213> Homo sapiens

<400> 3
atgaactttc tgctgtcttg ggtgcattgg agccttgcct tgctgctcta cctccaccat 60
gccaagtggt cccaggctgc acccatggca gaaggaggag ggcagaatca tcacgaagtg 120
gtgaagttca tggatgtcta tcagcgcagc tactgccatc caatcgagac cctggtggac 180
atcttccagg agtaccctga tgagatcgag tacatcttca agccatcctg tgtgcccctg 240
atgcgatgcg ggggctgctg caatgacgag ggcctggagt gtgtgcccac tgaggagtcc 300
aacatcacca tgcagattat gcggatcaaa cctcaccaag gccagcacat aggagagatg 360
agcttcctac agcacaacaa atgtgaatgc agaccaaaga aagatagagc aagacaagaa 420
aatccctgtg ggccttgctc agagcggaga aagcatttgt ttgtacaaga tccgcagacg 480
tgtaaatgtt cctgcaaaaa cacagactcg cgttgcaagg cgaggcagct tgagttaaac 540
gaacgtactt gcagatgtga caagccgagg cggtga 576

<210> 4
<211> 648
<212> DNA
<213> Homo sapiens

<400> 4
atgaactttc tgctgtcttg ggtgcattgg agccttgcct tgctgctcta cctccaccat 60
gccaagtggt cccaggctgc acccatggca gaaggaggag ggcagaatca tcacgaagtg 120
gtgaagttca tggatgtcta tcagcgcagc tactgccatc caatcgagac cctggtggac 180
atcttccagg agtaccctga tgagatcgag tacatcttca agccatcctg tgtgcccctg 240
atgcgatgcg ggggctgctg caatgacgag ggcctggagt gtgtgcccac tgaggagtcc 300
aacatcacca tgcagattat gcggatcaaa cctcaccaag gccagcacat aggagagatg 360
agcttcctac agcacaacaa atgtgaatgc agaccaaaga aagatagagc aagacaagaa 420
aaaaaatcag ttcgaggaaa gggaaagggg caaaaacgaa agcgcaagaa atcccggtat 480
aagtcctgga gcgttccctg tgggccttgc tcagagcgga gaaagcattt gtttgtacaa 540
gatccgcaga cgtgtaaatg ttcctgcaaa aacacagact cgcgttgcaa ggcgaggcag 600
cttgagttaa acgaacgtac ttgcagatgt gacaagccga ggcggtga 648

<210> 5
<211> 670
<212> DNA
<213> Homo sapiens

<400> 5
gccttgctgc tctacctcca ccatgccaag tggtcccagg ctgcacccat ggcagaagga 60
ggagggcaga atcatcacga agtggtgaag ttcatggatg tctatcagcg cagctactgc 120
catccaatcg agaccctggt ggacatcttc caggagtacc ctgatgagat cgagtacatc 180
ttcaagccat cctgtgtgcc cctgatgcga tgcgggggct gctgcaatga cgagggcctg 240
gagtgtgtgc ccactgagga gtccaacatc accatgcaga ttatgcggat caaacctcac 300
caaggccagc acataggaga gatgagcttc ctacagcaca acaaatgtga atgcagacca 360
aagaaggata gagcaagaca agaaaaaaaa tcagttcgag gaaagggaaa ggggcaaaaa 420
cgaaagcgca agaaatcccg gtataagtcc tggagcgttt acgttggtgc ccgctgctgt 480
ctaatgccct ggagcctccc tggcccccat ccctgtgggc cttgctcaga gcggagaaag 540
catttgtttg tacaagatcc gcagacgtgt aaatgttcct gcaaaaacac agactcgcgt 600

tgcaaggcga ggcagcttga gttaaacgaa cgtacttgca gatgtgacaa gccgaggcgg 660
tgatgaatga 670

<210> 6
<211> 1137
<212> DNA
<213> Homo sapiens

<400> 6
atgctcattg tccagactgg ggtcagatca gcaaacaaag ggcctctgat ggtgattgtt 60
gaatattgca aatatggaaa tctatccaac tacctcaaga gcaaatatga cttatttttt 120
ctcgacaagg atgtggcatc acacatggag cgtaagaaag aaaaaatgga gccaggcctg 180
gaacaaggca agaaaccaaa actagatagc atcaccagca gcgagagctt tgggagctcc 240
aagtttcagg aagataaaaa tctgagtgat gttgaggaag aggaggattc tgatggtttc 300
taccaggagc ccatcactat ggaagatctg atttcttaca gttttcaagt ggccagaggc 360
atgaagtttc tgtcttccag aaagtgcatt cattgggacc tggcagcaag aaacattctt 420
ttatctgaga acaatgtggt gaagatttgt gattttggcc ttgcccagga tatttacaag 480
aacgccgatt atgtgagaaa aggaggtggg tctccatacc caggagtgca aatggatgag 540
cacttctgca gttgcctgag ggaaggcatg aggatgagag ctgctgagta ctccactcct 600
gaaatctatc agatcatgct ggactgcagg cacaaagacc caaaagaaag gccaagattt 660
gcagaacttg tggaaaaact agaaaatagt gggtttacat actcaactcc tgccttctct 720
gaggacttct tcaaggaagg tatttcagct cccaagttta gttcaggaag ctctgatgat 780
gtcagatacg taaatgcttt caagttcatg agcctggaaa gaatcaaaac ctttgaagaa 840
cttttgccaa atgccacctc catgtttgat gactaccagg gggacagcag cgctctgctg 900
gcctctccca tgctgaagcg cttcaccagg actgacagca aacccaaggc ctcgctcaag 960
attgacttga gactaactag caaaagtaag aagtcggggc tttctgatgt cagcaggccc 1020
agtttctgcc attccaacag tgggcacatc agcaaaggca agggcaggtt cacctacgac 1080
aacgccgagc tggaaaggaa gacggcgtgc tgctccccgc ccctctggga gttgtag 1137

<210> 7
<211> 5830
<212> DNA
<213> Homo sapiens

<400> 7
actgagtccc gggaccccgg gagagcggtc agtgtgtggt cgctgcgttt cctctgcctg 60
cgccgggcat cacttgcgcg ccgcagaaag tccgtctggc agcctggata tcctctccta 120
ccggcacccg cagacgcccc tgcagccgcc ggtcggcgcc cgggctccct agccctgtgc 180
gctcaactgt cctgcgctgc ggggtgccgc gagttccacc tccgcgcctc cttctctaga 240
caggcgctgg gagaaagaac cggctcccga gttctgggca tttcgcccgg ctcgaggtgc 300
aggatgcaga gcaaggtgct gctggccgtc gccctgtggc tctgcgtgga gacccgggcc 360
gcctctgtgg gtttgcctag tgtttctctt gatctgccca ggctcagcat acaaaaagac 420
atacttacaa ttaaggctaa tacaactctt caaattactt gcaggggaca gagggacttg 480
gactggcttt ggcccaataa tcagagtggc agtgagcaaa gggtggaggt gactgagtgc 540
agcgatggcc tcttctgtaa gacactcaca attccaaaag tgatcggaaa tgacactgga 600
gcctacaagt gcttctaccg ggaaactgac ttggcctcgg tcatttatgt ctatgttcaa 660
gattacagat ctccatttat tgcttctgtt agtgaccaac atggagtcgt gtacattact 720
gagaacaaaa acaaaactgt ggtgattcca tgtctcgggt ccatttcaaa tctcaacgtg 780
tcactttgtg caagataccc agaaaagaga tttgttcctg atggtaacag aatttcctgg 840
gacagcaaga agggctttac tattcccagc tacatgatca gctatgctgg catggtcttc 900
tgtgaagcaa aaattaatga tgaaagttac cagtctatta tgtacatagt tgtcgttgta 960
gggtatagga tttatgatgt ggttctgagt ccgtctcatg gaattgaact atctgttgga 1020
gaaaagcttg tcttaaattg tacagcaaga actgaactaa atgtggggat tgacttcaac 1080
tgggaatacc cttcttcgaa gcatcagcat aagaaacttg taaaccgaga cctaaaaacc 1140
cagtctggga gtgagatgaa gaaatttttg agcaccttaa ctatagatgg tgtaacccgg 1200
agtgaccaag gattgtacac ctgtgcagca tccagtgggc tgatgaccaa gaagaacagc 1260
acatttgtca gggtccatga aaaacctttt gttgcttttg gaagtggcat ggaatctctg 1320
gtggaagcca cggtggggga gcgtgtcaga atccctgcga agtaccttgg ttacccaccc 1380
ccagaaataa aatggtataa aaatggaata ccccttgagt ccaatcacac aattaaagcg 1440
gggcatgtac tgacgattat ggaagtgagt gaaagagaca caggaaatta cactgtcatc 1500
cttaccaatc ccatttcaaa ggagaagcag agccatgtgg tctctctggt tgtgtatgtc 1560
ccaccccaga ttggtgagaa atctctaatc tctcctgtgg attcctacca gtacggcacc 1620
actcaaacgc tgacatgtac ggtctatgcc attcctcccc cgcatcacat ccactggtat 1680
tggcagttgg aggaagagtg cgccaacgag cccagccaag ctgtctcagt gacaaaccca 1740
tacccttgtg aagaatggag aagtgtggag gacttccagg gaggaaataa aattgaagtt 1800
aataaaaatc aatttgctct aattgaagga aaaaacaaaa ctgtaagtac ccttgttatc 1860
caagcggcaa atgtgtcagc tttgtacaaa tgtgaagcgg tcaacaaagt cgggagagga 1920
gagagggtga tctccttcca cgtgaccagg ggtcctgaaa ttactttgca acctgacatg 1980
cagcccactg agcaggagag cgtgtctttg tggtgcactg cagacagatc tacgtttgag 2040

aacctcacat ggtacaagct tggcccacag cctctgccaa tccatgtggg agagttgccc 2100
acacctgttt gcaagaactt ggatactctt tggaaattga atgccaccat gttctctaat 2160
agcacaaatg acattttgat catggagctt aagaatgcat ccttgcagga ccaaggagac 2220
tatgtctgcc ttgctcaaga caggaagacc aagaaaagac attgcgtggt caggcagctc 2280
acagtcctag agcgtgtggc acccacgatc acaggaaacc tggagaatca gacgacaagt 2340
attggggaaa gcatcgaagt ctcatgcacg gcatctggga atccccctcc acagatcatg 2400
tggtttaaag ataatgagac ccttgtagaa gactcaggca ttgtattgaa ggatgggaac 2460
cggaacctca ctatccgcag agtgaggaag gaggacgaag gcctctacac ctgccaggca 2520
tgcagtgttc ttggctgtgc aaaagtggag gcatttttca taatagaagg tgcccaggaa 2580
aagacgaact tggaaatcat tattctagta ggcacggcgg tgattgccat gttcttctgg 2640
ctacttcttg tcatcatcct acggaccgtt aagcgggcca atggagggga actgaagaca 2700
ggctacttgt ccatcgtcat ggatccagat gaactcccat tggatgaaca ttgtgaacga 2760
ctgccttatg atgccagcaa atgggaattc cccagagacc ggctgaagct aggtaagcct 2820
cttggccgtg gtgcctttgg ccaagtgatt gaagcagatg cctttggaat tgacaagaca 2880
gcaacttgca ggacagtagc agtcaaaatg ttgaaagaag gagcaacaca cagtgagcat 2940
cgagctctca tgtctgaact caagatcctc attcatattg gtcaccatct caatgtggtc 3000
aaccttctag gtgcctgtac caagccagga gggccactca tggtgattgt ggaattctgc 3060
aaatttggaa acctgtccac ttacctgagg agcaagagaa atgaatttgt cccctacaag 3120
accaaagggg cacgattccg tcaagggaaa gactacgttg gagcaatccc tgtggatctg 3180
aaacggcgct tggacagcat caccagtagc cagagctcag ccagctctgg atttgtggag 3240
gagaagtccc tcagtgatgt agaagaagag gaagctcctg aagatctgta taaggacttc 3300
ctgaccttgg agcatctcat ctgttacagc ttccaagtgg ctaagggcat ggagttcttg 3360
gcatcgcgaa agtgtatcca cagggacctg gcggcacgaa atatcctctt atcggagaag 3420
aacgtggtta aaatctgtga ctttggcttg gcccgggata tttataaaga tccagattat 3480
gtcagaaaag gagatgctcg cctccctttg aaatggatgg ccccagaaac aatttttgac 3540
agagtgtaca caatccagag tgacgtctgg tcttttggtg ttttgctgtg ggaaatattt 3600
tccttaggtg cttctccata tcctggggta aagattgatg aagaattttg taggcgattg 3660
aaagaaggaa ctagaatgag ggcccctgat tatactacac cagaaatgta ccagaccatg 3720
ctggactgct ggcacgggga gcccagtcag agacccacgt tttcagagtt ggtggaacat 3780
ttgggaaatc tcttgcaagc taatgctcag caggatggca aagactacat tgttcttccg 3840
atatcagaga ctttgagcat ggaagaggat tctggactct ctctgcctac ctcacctgtt 3900
tcctgtatgg aggaggagga agtatgtgac cccaaattcc attatgacaa cacagcagga 3960
atcagtcagt atctgcagaa cagtaagcga aagagccggc ctgtgagtgt aaaaacattt 4020
gaagatatcc cgttagaaga accagaagta aaagtaatcc cagatgacaa ccagacggac 4080
agtggtatgg ttcttgcctc agaagagctg aaaactttgg aagacagaac caaattatct 4140
ccatcttttg gtggaatggt gcccagcaaa agcagggagt ctgtggcatc tgaaggctca 4200
aaccagacaa gcggctacca gtccggatat cactccgatg acacagacac caccgtgtac 4260
tccagtgagg aagcagaact tttaaagctg atagagattg gagtgcaaac cggtagcaca 4320
gcccagattc tccagcctga ctcggggacc acactgagct ctcctcctgt ttaaaaggaa 4380
gcatccacac cccaactccc ggacatcaca tgagaggtct gctcagattt tgaagtgttg 4440
ttctttccac cagcaggaag tagccgcatt tgattttcat ttcgacaaca gaaaaaggac 4500
ctcggactgc agggagccag tcttctaggc atatcctgga agaggcttgt gacccaagaa 4560
tgtgtctgtg tcttctccca gtgttgacct gatcctcttt tttcattcat ttaaaaagca 4620
ttatcatgcc cctgctgcgg gtctcaccat gggtttagaa caaagagctt caagcaatgg 4680
ccccatcctc aaagaagtag cagtacctgg ggagctgaca cttctgtaaa actagaagat 4740
aaaccaggca acgtaagtgt tcgaggtgtt gaagatggga aggatttgca gggctgagtc 4800
tatccaagag gctttgttta ggacgtgggt cccaagccaa gccttaagtg tggaattcgg 4860
attgatagaa aggaagacta acgttacctt gctttggaga gtactggagc ctgcaaatgc 4920
attgtgtttg ctctggtgga ggtgggcatg gggtctgttc tgaaatgtaa agggttcaga 4980
cggggtttct ggttttagaa ggttgcgtgt tcttcgagtt gggctaaagt agagttcgtt 5040
gtgctgtttc tgactcctaa tgagagttcc ttccagaccg ttagctgtct ccttgccaag 5100
ccccaggaag aaaatgatgc agctctggct ccttgtctcc caggctgatc ctttattcag 5160
aataccacaa agaaaggaca ttcagctcaa ggctccctgc cgtgttgaag agttctgact 5220
gcacaaacca gcttctggtt tcttctggaa tgaataccct catatctgtc ctgatgtgat 5280
atgtctgaga ctgaatgcgg gaggttcaat gtgaagctgt gtgtggtgtc aaagtttcag 5340
gaaggatttt acccttttgt tcttccccct gtccccaacc cactctcacc ccgcaaccca 5400
tcagtatttt agttatttgg cctctactcc agtaaacctg attgggtttg ttcactctct 5460
gaatgattat tagccagact tcaaaattat tttatagccc aaattataac atctattgta 5520
ttatttagac ttttaacata tagagctatt tctactgatt tttgcccttg ttctgtcctt 5580
tttttcaaaa aagaaaatgt gttttttgtt tggtaccata gtgtgaaatg ctgggaacaa 5640
tgactataag acatgctatg gcacatatat ttatagtctg tttatgtaga aacaaatgta 5700
atatattaaa gccttatata taatgaactt tgtactattc acattttgta tcagtattat 5760
gtagcataac aaaggtcata atgctttcag caattgatgt cattttatta aagaacattg 5820
aaaaacttga 5830

<210> 8
<211> 19

<212> DNA
<213> Artificial Sequence

<220>
<223> Targeting Sequence

<400> 8
tcatcacgaa gtggtgaag                                                    19

<210> 9
<211> 21
<212> RNA
<213> Artificial Sequence

<220>
<223> Sense strand

<400> 9
ucaucacgaa guggugaagu u                                                 21

<210> 10
<211> 21
<212> RNA
<213> Artificial Sequence

<220>
<223> Antisense strand

<400> 10
cuucaccacu ucgugaugau u                                                 21

<210> 11
<211> 21
<212> DNA
<213> Artificial Sequence

<220>
<223> Sense strand

<221> misc_RNA
<222> (1)...(19)
<223> ribonucleotide

<221> misc_feature
<222> (20)...(21)
<223> deoxyribonucleotide

<400> 11
ucaucacgaa guggugaagt t                                                 21

<210> 12
<211> 21
<212> DNA
<213> Artificial Sequence

<220>
<223> Antisense strand

<221> misc_RNA
<222> (1)...(19)
<223> ribonucleotides

<221> misc_feature
<222> (20)...(21)
<223> deoxyribonucleotides

<400> 12
cuucaccacu ucgugaugat t                                                 21

<210> 13
<211> 21
<212> DNA
<213> Artificial Sequence

<220>
<223> Targeting Sequence

<400> 13
aacgtacttg cagatgtgac a                                                21

<210> 14
<211> 19
<212> DNA
<213> Artificial Sequence

<220>
<223> Targeting Sequence

<400> 14
gttcatggat gtctatcag                                                   19

<210> 15
<211> 19
<212> DNA
<213> Artificial Sequence

<220>
<223> Targeting Sequence

<400> 15
tcgagaccct ggtggacat                                                   19

<210> 16
<211> 19
<212> DNA
<213> Artificial Sequence

<220>
<223> Targeting Sequence

<400> 16
tgacgagggc ctggagtgt                                                   19

<210> 17
<211> 19
<212> DNA
<213> Artificial Sequence

<220>
<223> Targeting Sequence

<400> 17
tgacgagggc ctggagtgt                                                   19

<210> 18
<211> 19
<212> DNA
<213> Artificial Sequence

<220>
<223> Targeting Sequence

<400> 18
catcaccatg cagattatg                                                   19

<210> 19
<211> 19
<212> DNA

<213> Artificial Sequence

<220>
<223> Targeting Sequence

<400> 19
acctcaccaa ggccagcac                                                    19

<210> 20
<211> 19
<212> DNA
<213> Artificial Sequence

<220>
<223> Targeting Sequence

<400> 20
ggccagcaca taggagaga                                                    19

<210> 21
<211> 19
<212> DNA
<213> Artificial Sequence

<220>
<223> Targeting Sequence

<400> 21
caaatgtgaa tgcagacca                                                    19

<210> 22
<211> 19
<212> DNA
<213> Artificial Sequence

<220>
<223> Targeting Sequence

<400> 22
atgtgaatgc agaccaaag                                                    19

<210> 23
<211> 19
<212> DNA
<213> Artificial Sequence

<220>
<223> Targeting Sequence

<400> 23
tgcagaccaa agaaagata                                                    19

<210> 24
<211> 19
<212> DNA
<213> Artificial Sequence

<220>
<223> Targeting Sequence

<400> 24
agaaagatag agcaagaca                                                    19

<210> 25
<211> 19
<212> DNA
<213> Artificial Sequence

<220>

<223> Targeting Sequence

<400> 25
gaaagataga gcaagacaa                                                    19

<210> 26
<211> 19
<212> DNA
<213> Artificial Sequence

<220>
<223> Targeting Sequence

<400> 26
gatagagcaa gacaagaaa                                                    19

<210> 27
<211> 19
<212> DNA
<213> Artificial Sequence

<220>
<223> Targeting Sequence

<400> 27
gacaagaaaa tccctgtgg                                                    19

<210> 28
<211> 19
<212> DNA
<213> Artificial Sequence

<220>
<223> Targeting Sequence

<400> 28
gaaaatccct gtgggcctt                                                    19

<210> 29
<211> 19
<212> DNA
<213> Artificial Sequence

<220>
<223> Targeting Sequence

<400> 29
aatccctgtg ggccttgct                                                    19

<210> 30
<211> 19
<212> DNA
<213> Artificial Sequence

<220>
<223> Targeting Sequence

<400> 30
tccctgtggg ccttgctca                                                    19

<210> 31
<211> 19
<212> DNA
<213> Artificial Sequence

<220>
<223> Targeting Sequence

<400> 31

gcatttgttt gtacaagat                                                    19

<210> 32
<211> 19
<212> DNA
<213> Artificial Sequence

<220>
<223> Targeting Sequence

<400> 32
gatccgcaga cgtgtaaat                                                    19

<210> 33
<211> 19
<212> DNA
<213> Artificial Sequence

<220>
<223> Targeting Sequence

<400> 33
atgttcctgc aaaaacaca                                                    19

<210> 34
<211> 19
<212> DNA
<213> Artificial Sequence

<220>
<223> Targeting Sequence

<400> 34
tgttcctgca aaaacacag                                                    19

<210> 35
<211> 19
<212> DNA
<213> Artificial Sequence

<220>
<223> Targeting Sequence

<400> 35
aaacacagac tcgcgttgc                                                    19

<210> 36
<211> 19
<212> DNA
<213> Artificial Sequence

<220>
<223> Targeting Sequence

<400> 36
aacacagact cgcgttgca                                                    19

<210> 37
<211> 19
<212> DNA
<213> Artificial Sequence

<220>
<223> Targeting Sequence

<400> 37
acacagactc gcgttgcaa                                                    19

<210> 38

<211> 19
<212> DNA
<213> Artificial Sequence

<220>
<223> Targeting Sequence

<400> 38
cacagactcg cgttgcaag                                                    19

<210> 39
<211> 19
<212> DNA
<213> Artificial Sequence

<220>
<223> Targeting Sequence

<400> 39
ggcgaggcag cttgagtta                                                    19

<210> 40
<211> 19
<212> DNA
<213> Artificial Sequence

<220>
<223> Targeting Sequence

<400> 40
acgaacgtac ttgcagatg                                                    19

<210> 41
<211> 19
<212> DNA
<213> Artificial Sequence

<220>
<223> Targeting Sequence

<400> 41
cgaacgtact tgcagatgt                                                    19

<210> 42
<211> 19
<212> DNA
<213> Artificial Sequence

<220>
<223> Targeting Sequence

<400> 42
cgtacttgca gatgtgaca                                                    19

<210> 43
<211> 19
<212> DNA
<213> Artificial Sequence

<220>
<223> Targeting Sequence

<400> 43
gtggtcccag gctgcaccc                                                    19

<210> 44
<211> 19
<212> DNA
<213> Artificial Sequence

<220>
<223> Targeting Sequence

<400> 44
ggaggagggc agaatcatc                                                    19

<210> 45
<211> 19
<212> DNA
<213> Artificial Sequence

<220>
<223> Targeting Sequence

<400> 45
gtggtgaagt tcatggatg                                                    19

<210> 46
<211> 21
<212> DNA
<213> Artificial Sequence

<220>
<223> Targeting Sequence

<400> 46
aatcatcacg aagtggtgaa g                                                 21

<210> 47
<211> 21
<212> DNA
<213> Artificial Sequence

<220>
<223> Targeting Sequence

<400> 47
aagttcatgg atgtctatca g                                                 21

<210> 48
<211> 21
<212> DNA
<213> Artificial Sequence

<220>
<223> Targeting Sequence

<400> 48
aatcgagacc ctggtggaca t                                                 21

<210> 49
<211> 21
<212> DNA
<213> Artificial Sequence

<220>
<223> Targeting Sequence

<400> 49
aatgacgagg gcctggagtg t                                                 21

<210> 50
<211> 21
<212> DNA
<213> Artificial Sequence

<220>
<223> Targeting Sequence

<400> 50
aacatcacca tgcagattat g                                                21

<210> 51
<211> 21
<212> DNA
<213> Artificial Sequence

<220>
<223> Targeting Sequence

<400> 51
aaacctcacc aaggccagca c                                                21

<210> 52
<211> 21
<212> DNA
<213> Artificial Sequence

<220>
<223> Targeting Sequence

<400> 52
aaggccagca cataggagag a                                                21

<210> 53
<211> 21
<212> DNA
<213> Artificial Sequence

<220>
<223> Targeting Sequence

<400> 53
aacaaatgtg aatgcagacc a                                                21

<210> 54
<211> 21
<212> DNA
<213> Artificial Sequence

<220>
<223> Targeting Sequence

<400> 54
aaatgtgaat gcagaccaaa g                                                21

<210> 55
<211> 21
<212> DNA
<213> Artificial Sequence

<220>
<223> Targeting Sequence

<400> 55
aatgcagacc aaagaaagat a                                                21

<210> 56
<211> 21
<212> DNA
<213> Artificial Sequence

<220>
<223> Targeting Sequence

<400> 56
aaagaaagat agagcaagac a                                                21

<210> 57
<211> 21
<212> DNA
<213> Artificial Sequence

<220>
<223> Targeting Sequence

<400> 57
aagaaagata gagcaagaca a                                                21

<210> 58
<211> 23
<212> DNA
<213> Artificial Sequence

<220>
<223> Targeting Sequence

<400> 58
aagatagagc aagacaagaa aat                                              23

<210> 59
<211> 23
<212> DNA
<213> Artificial Sequence

<220>
<223> Targeting Sequence

<400> 59
aagacaagaa aatccctgtg ggc                                              23

<210> 60
<211> 23
<212> DNA
<213> Artificial Sequence

<220>
<223> Targeting Sequence

<400> 60
aagaaaatcc ctgtgggcct tgc                                              23

<210> 61
<211> 23
<212> DNA
<213> Artificial Sequence

<220>
<223> Targeting Sequence

<400> 61
aatccctgtg ggccttgctc aga                                              23

<210> 62
<211> 23
<212> DNA
<213> Artificial Sequence

<220>
<223> Targeting Sequence

<400> 62
aagcatttgt ttgtacaaga tcc                                              23

<210> 63
<211> 23

<212> DNA
<213> Artificial Sequence

<220>
<223> Targeting Sequence

<400> 63
aagatccgca gacgtgtaaa tgt                                              23

<210> 64
<211> 23
<212> DNA
<213> Artificial Sequence

<220>
<223> Targeting Sequence

<400> 64
aaatgttcct gcaaaaacac aga                                              23

<210> 65
<211> 23
<212> DNA
<213> Artificial Sequence

<220>
<223> Targeting Sequence

<400> 65
aatgttcctg caaaaacaca gac                                              23

<210> 66
<211> 23
<212> DNA
<213> Artificial Sequence

<220>
<223> Targeting Sequence

<400> 66
aaaaacacag actcgcgttg caa                                              23

<210> 67
<211> 23
<212> DNA
<213> Artificial Sequence

<220>
<223> Targeting Sequence

<400> 67
aaaacacaga ctcgcgttgc aag                                              23

<210> 68
<211> 23
<212> DNA
<213> Artificial Sequence

<220>
<223> Targeting Sequence

<400> 68
aaacacagac tcgcgttgca agg                                              23

<210> 69
<211> 23
<212> DNA
<213> Artificial Sequence

<220>
<223> Targeting Sequence

<400> 69
aacacagact cgcgttgcaa ggc                                               23

<210> 70
<211> 23
<212> DNA
<213> Artificial Sequence

<220>
<223> Targeting Sequence

<400> 70
aaggcgaggc agcttgagtt aaa                                               23

<210> 71
<211> 23
<212> DNA
<213> Artificial Sequence

<220>
<223> Targeting Sequence

<400> 71
aaacgaacgt acttgcagat gtg                                               23

<210> 72
<211> 23
<212> DNA
<213> Artificial Sequence

<220>
<223> Targeting Sequence

<400> 72
aacgaacgta cttgcagatg tga                                               23

<210> 73
<211> 23
<212> DNA
<213> Artificial Sequence

<220>
<223> Targeting Sequence

<400> 73
aagtggtccc aggctgcacc cat                                               23

<210> 74
<211> 23
<212> DNA
<213> Artificial Sequence

<220>
<223> Targeting Sequence

<400> 74
aaggaggagg gcagaatcat cac                                               23

<210> 75
<211> 23
<212> DNA
<213> Artificial Sequence

<220>
<223> Targeting Sequence

<400> 75
aagtggtgaa gttcatggat gtc                                              23

<210> 76
<211> 23
<212> DNA
<213> Artificial Sequence

<220>
<223> Targeting Sequence

<400> 76
aaaatccctg tgggccttgc tca                                              23

<210> 77
<211> 21
<212> DNA
<213> Artificial Sequence

<220>
<223> Sense strand

<221> misc_RNA
<222> (1)...(19)
<223> ribonucleotides

<221> misc_feature
<222> (20)...(21)
<223> deoxyribonucleotides

<400> 77
accucaccaa ggccagcact t                                                21

<210> 78
<211> 21
<212> DNA
<213> Artificial Sequence

<220>
<223> Antisense strand

<221> misc_RNA
<222> (1)...(19)
<223> ribonucleotides

<221> misc_feature
<222> (20)...(21)
<223> deoxyribonucleotides

<400> 78
gugcuggccu uggugaggut t                                                21

<210> 79
<211> 18
<212> DNA
<213> Artificial Sequence

<220>
<223> Targeting sequence

<400> 79
ggctacgtcc agcgcacc                                                    18

<210> 80
<211> 21
<212> DNA
<213> Artificial Sequence

<220>

<223> Targeting sequence

<400> 80
aaaccucacc aaagccagca c                                                21

<210>  81
<211>  21
<212>  DNA
<213>  Artificial

<220>
<223>  Targeting Sequence

<400>  81
aaggaggagg gcagaatcat c                                                21

<210>  82
<211>  21
<212>  DNA
<213>  Artificial

<220>
<223>  Targeting Sequence

<400>  82
aatgtgaatg cagaccaaag a                                                21

<210>  83
<211>  21
<212>  DNA
<213>  Artificial

<220>
<223>  Targeting Sequence

<400>  83
aaagcatttg tttgtacaag a                                                21

<210>  84
<211>  21
<212>  DNA
<213>  Artificial

<220>
<223>  Targeting Sequence

<400>  84
aagatccgca gacgtgtaaa t                                                21

<210>  85
<211>  21
<212>  DNA
<213>  Artificial

<220>
<223>  Targeting Sequence

<400>  85
aaacacacac tcgcgttgca a                                                21

## Description

### B. CROSS REFERENCE TO RELATED APPLICATION

This application claims the benefit of U.S. Provisional Application No. 60/804,500 entitled "Compositions and Methods for siRNA Inhibition of VEGF in the Eye", filed on June 12, 2006, U.S. Provisional Application No. 60/824,972 entitled "Compositions and Methods for siRNA Inhibition of VEGF in the Eye", filed on September 8, 2006 and U.S. Provisional Application No. 60/864,440 entitled "Compositions and Methods for siRNA Inhibition of VEGF in the Eye", filed on November 6, 2006, herein incorporated by reference in their entireties.

### C. REFERENCE TO GOVERNMENT GRANT- NOT APPLICABLE

### D. JOINT RESEARCH AGREEMENT - NOT APPLICABLE

### E. FIELD OF THE INVENTION - NOT APPLICABLE

### F. BACKGROUND OF THE INVENTION - NOT APPLICABLE

### G. SUMMARY OF THE INVENTION

Embodiments of the present invention provide methods of stabilizing visual acuity comprising administering to a subject an effective amount of an siRNA targeting VEGF. Preferably, the siRNA comprises a sense RNA strand of SEQ ID NO: 77 and an antisense RNA strand of SEQ ID NO: 78.

Further embodiments of the present invention provide methods of inhibiting choroidal neovascularization comprising administering to a subject an effective amount of an siRNA targeting VEGF. Preferably, the siRNA comprises a sense RNA strand of SEQ ID NO: 77 and an antisense RNA strand of SEQ ID NO: 78.

Other embodiments of the present invention provide methods of treating diabetic macular edema comprising administering to a subject an effective amount of an siRNA targeting VEGF. Preferably, the siRNA comprises a sense RNA strand of SEQ ID NO: 77 and an antisense RNA strand of SEQ ID NO: 78.

Additional embodiments of the present invention provide methods of decreasing foveal thickness comprising administering to a subject an effective amount of an siRNA targeting VEGF. Preferably, the siRNA comprises a sense RNA strand of SEQ ID NO: 77 and an antisense RNA strand of SEQ ID NO: 78.

Further embodiments of the present invention provide methods of treating macular degeneration, particularly age-related macular degeneration, comprising administering an siRNA targeting VEGF and a VEGF antagonist. Preferably, the siRNA comprises a sense RNA strand of SEQ ID NO: 77 and an antisense RNA strand of SEQ ID NO: 78.

Additional embodiments of the present invention provide methods of treating diabetic macular edema comprising administering an siRNA targeting VEGF and a VEGF antagonist. Preferably, the siRNA comprises a sense RNA strand of SEQ ID NO: 77 and an antisense RNA strand of SEQ ID NO: 78.

Additional embodiments of the present invention provide methods of treating diabetic retinopathy comprising administering an siRNA targeting VEGF. Preferably, the siRNA comprises a sense RNA strand of SEQ ID NO: 77 and an antisense RNA strand of SEQ ID NO: 78.

Additional embodiments of the present invention provide methods of treating proliferative diabetic retinopathy comprising administering an siRNA targeting VEGF. Preferably, the siRNA comprises a sense RNA strand of SEQ ID NO: 77 and an antisense RNA strand of SEQ ID NO: 78.

In a further embodiment of the present invention, pharmaceutical compositions comprising an siRNA comprising a sense RNA strand of SEQ ID NO: 77 and an antisense RNA strand of SEQ ID NO: 78 are provided.

### H. BRIEF DESCRIPTION OF THE DRAWINGS

The file of this patent contains at least one photograph or drawing executed in color. Copies of this patent with color drawing(s) or photograph(s) will be provided by the Patent and Trademark Office upon request and payment of necessary fee.

For a fuller understanding of the nature and advantages of the present invention, reference should be had to the following detailed description taken in connection with the accompanying drawings, in which:

**Fig. 1** is a graph of hVEGF protein levels in 293 cells transfected with human VEGF siRNAs, non-specific siRNA (EGFP siRNA) or mock transfections without siRNA.

**Fig. 2** is a graph of the dose response studies with Cand5, HVEGF#1, hVEGF#2, hVEGF#3, hVEGF#4, hVEGF#6 and hVEGF#7.

**Fig. 3** is a graph of the mean change in best corrected distance visual acuity in human subjects as measured by the early treatment diabetic retinopathy study ("ETDRS") methodology in each of the three study groups receiving different doses of Cand5.

**Fig. 4** is a pair of histograms showing the percentage of human subjects in each of the study groups that exhibited stabilization of distance visual acuity as determined by the loss of fewer than 15 letters in the ETDRS test at 12 weeks compared to baseline, or at 15 weeks as compared to 3 weeks.

**Fig. 5** is a graph of the mean change in best corrected near visual acuity in human subjects as measured by the ETDRS methodology in each of the three study groups receiving different doses of Cand5.

**Fig. 6** is a pair of histograms showing the percentage of human subjects in each of the study groups that exhibited stabilization of near visual acuity as determined by the loss of fewer than 15 letters in the ETDRS test at 12 weeks compared to baseline, or at 15 weeks as compared to 3 weeks.

**Figs. 7A** **and** **7B** are a pair of graphs of the mean change in best corrected distance visual acuity in each of the three Cand5 study groups compared to the control placebo group of the related TAP study. Fig. 7B applies Last Observed Carried Forward ("LOCF") analysis to the data sets.

**Figs. 8A** **and** **8B** are a pair of graphs of the change from baseline in mean size of the Choroidal Neovascularization (CNV) lesions in each of the three Cand5 study groups, Fig. 8B applies LOCF analysis to the data sets.

**Fig. 9** is a graph of the change in foveal thickness as measured by ocular coherence tomography ("OCT") in three groups of human subjects with DME treated with different doses of Cand5.

**Fig. 10** is a graph of the change in visual acuity as measured by ETDRS in three groups of human subjects with DME treated with different doses of Cand5.

### I. DETAILED DESCRIPTION OF THE INVENTION

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. All publications mentioned herein are incorporated by reference in their entirety. Nothing herein is to be construed as an admission that the invention is not entitled to antedate such disclosure by virtue of prior invention.

It must also be noted that as used herein and in the appended claims, the singular forms "a", "an", and "the" include plural reference unless the context clearly dictates otherwise. Thus, for example, reference to a "molecule" is a reference to one or more molecules and equivalents thereof known to those skilled in the art, and so forth.

As used herein, the term "about" means plus or minus 10% of the numerical value of the number with which it is being used. therefore, about 50% means in the range of 45%-55%.

As used herein, a "subject" includes a human being or non-human animal. Preferably, the subject is a human being.

As used herein, an "effective amount" of the siRNA is an amount sufficient to cause RNAi-mediated degradation of the target mRNA, or an amount sufficient to inhibit the progression of angiogenesis in a subject.

As used herein, "isolated" means altered or removed from the natural state through human intervention. For example, an siRNA naturally present in a living animal is not "isolated," but a synthetic siRNA, or an siRNA partially or completely separated from the coexisting materials of its natural state is "isolated." An isolated siRNA can exist in substantially purified form, or can exist in a non-native environment such as, for example, a cell into which the siRNA has been delivered.

As used herein, "target mRNA" means human VEGF mRNA.

Unless otherwise indicated, all nucleic acid sequences herein are given in the 5' to 3' direction. Also, all deoxyribonucleotides in a nucleic acid sequence are represented by capital letters (*e.g.*, deoxythymidine is "T"), and ribonucleotides in a nucleic acid sequence are represented by lower case letters (*e.g.*, uridine is "u").

Angiogenesis, defined as the growth of new capillary blood vessels or "neovascularization," plays a fundamental role in growth and development. In mature humans, the ability to initiate angiogenesis is present in all tissues, but is held under strict control. A key regulator of angiogenesis is vascular endothelial growth factor ("VEGF"), also called vascular permeability factor ("VPF"). VEGF exists in at least four different alternative splice forms in humans (VEGF₁₂₁, VEGF₁₆₅, VEGF₁₈₉ and VEGF₂₀₆), all of which exert similar biological activities.

Angiogenesis is initiated when secreted VEGF binds to the Flt-1 and Flk-1/KDR receptors (also called VEGF receptor 1 and VEGF receptor 2), which are expressed on the surface of endothelial cells. Flt-1 and Flk-1/KDR are transmembrane protein tyrosine kinases, and binding of VEGF initiates a cell signal cascade resulting ultimately in the neovascularization of the surrounding tissue.

Aberrant angiogenesis, or the pathogenic growth of new blood vessels, is implicated in a number of conditions. Among these conditions are diabetic retinopathy, diabetic macular edema ("DME"), psoriasis, exudative or "wet" age-related macular degeneration ("ARMD"), rheumatoid arthritis and other inflammatory diseases, and most cancers. The diseases associated with these conditions exhibit abnormally high levels of VEGF, and generally show a high degree of vascularization or vascular permeability.

ARMD in particular is a clinically important angiogenic disease. This condition is characterized by choroidal neovascularization in one or both eyes in aging individuals, and is the major cause of blindness in industrialized countries.

Diabetic macular edema (DME), also called diabetic retinopathy, is a complication of the chronically high blood sugar afflicting diabetics. It is caused by leakiness of retinal blood vessels and the growth of new blood vessels on the retina, optic nerve and the iris. The leaky blood vessels result in swelling of the retina and visual loss. The new blood vessels that grow on the optic nerve and retina can also bleed, resulting in severe visual loss. In addition, new blood vessels in the iris clog the drain of the eye and can result in extremely high pressure in the eye with accompanying intense pain and the potential loss of the eye. DME can affect almost anyone with diabetes. In general, the longer someone has diabetes, the greater the risk of developing DME. Eventually, almost everyone with juvenile-onset diabetes will develop some symptoms of DME. Those who acquire diabetes later in life are also at risk of DME, although they are somewhat less likely to develop advanced DME.

A number of therapeutic strategies exist for inhibiting aberrant angiogenesis, which attempt to reduce the production or effect of VEGF, otherwise referred to herein as "VEGF antagonists". For example, anti-VEGF or anti-VEGF receptor antibodies, anti-VEGF aptamers, and soluble VEGF traps, which compete with endothelial cell receptors for VEGF binding have been developed. Classical VEGF "antisense" therapies directed against VEGF gene expression have also been proposed. However, the anti-angiogenic agents used in these therapies can produce only a stoichiometric reduction in VEGF or VEGF receptor, and the agents are typically overwhelmed by the abnormally high production of VEGF by the diseased tissue. The results achieved with available anti-angiogenic therapies have therefore been unsatisfactory.

RNA interference (hereinafter "RNAi") is a method of post-transcriptional gene regulation that is conserved throughout many eukaryotic organisms. RNAi is induced by short (*i.e.*, <30 nucleotide) double stranded RNA ("dsRNA") molecules which are present in the cell. These short dsRNA molecules, called "short interfering RNA" or "siRNA," cause the destruction of messenger RNAs ("mRNAs") which share sequence homology with the siRNA to within one nucleotide resolution. It is believed that the siRNA and the targeted mRNA bind to an "RNA-induced silencing complex" or "RISC", which cleaves the targeted mRNA. The siRNA is apparently recycled much like a multiple-turnover enzyme, with 1 siRNA molecule capable of inducing cleavage of approximately 1000 mRNA molecules. siRNA-mediated RNAi degradation of an mRNA is therefore more effective than currently available technologies for inhibiting expression of a target gene.

One embodiment of the present invention therefore provides isolated siRNA comprising short double-stranded RNA from about 17 nucleotides to about 29 nucleotides in length, preferably from about 19 to about 25 nucleotides in length, that are targeted to the target mRNA. The siRNA comprise a sense RNA strand and a complementary antisense RNA strand annealed together by standard Watson-Crick base-pairing interactions (hereinafter "base-paired"). As is described in more detail below, the sense strand comprises a nucleic acid sequence which is identical to a target sequence contained within the target mRNA.

The sense and antisense strands of the siRNA can comprise two complementary, single-stranded RNA molecules or can comprise a single molecule in which two complementary portions are base-paired and are covalently linked, for example, by a single-stranded "hairpin" loop. Without wishing to be bound by any theory, it is believed that the hairpin loop of the latter type of siRNA molecule is cleaved intracellularly by the "Dicer" protein (or its equivalent) to form an siRNA of two individual base-paired RNA molecules.

Splice variants of human VEGF are known, including VEGF₁₂₁ (SEQ ID NO: 2), VEGF₁₆₅ (SEQ ID NO: 3), VEGF₁₈₉ (SEQ ID NO: 4) and VEGF₂₀₆ (SEQ ID NO: 5). The mRNA transcribed from the human VEGF, Flt-1 (SEQ ID NO: 6) or Flk-1/KDR (SEQ ID NO: 7) genes can be analyzed for further alternative splice forms using techniques well-known in the art. Such techniques include reverse transcription-polymerase chain reaction (RT-PCR), northern blotting and in-situ hybridization. Techniques for analyzing mRNA sequences are described, for example, in Busting SA (2000), J. Mol. Endocrinol. 25: 169-193, the entire disclosure of which is herein incorporated by reference. Representative techniques for identifying alternatively spliced mRNAs are also described below.

For example, databases that contain nucleotide sequences related to a given disease gene can be used to identify alternatively spliced mRNA. Such databases include GenBank, Embase, and the Cancer Genome Anatomy Project (CGAP) database. The CGAP database, for example, contains expressed sequence tags (ESTs) from various types of human cancers. An mRNA or gene sequence from the VEGF gene can be used to query such a database to determine whether ESTs representing alternatively spliced mRNAs have been found for this gene.

A technique called "RNAse protection" can also be used to identify alternatively spliced VEGF mRNA. RNAse protection involves transcription of a gene sequence into synthetic RNA, which is hybridized to RNA derived from other cells; for example, cells from tissue at or near the site of neovascularization. The hybridized RNA is then incubated with enzymes that recognize RNA:RNA hybrid mismatches. Smaller than expected fragments indicate the presence of alternatively spliced mRNAs. The putative alternatively spliced mRNAs can be cloned and sequenced by methods well known to those skilled in the art.

RT-PCR can also be used to identify alternatively spliced VEGF mRNA. In RT-PCR, RNA from the diseased tissue is converted into cDNA by the enzyme reverse transcriptase, using methods well-known to those of ordinary skill in the art. The entire coding sequence of the cDNA is then amplified via PCR using a forward primer located in the 3' untranslated region, and a reverse primer located in the 5' untranslated region. The amplified products can be analyzed for alternative splice forms, for example by comparing the size of the amplified products with the size of the expected product from normally spliced mRNA, *e.g.*, by agarose gel electrophoresis. Any change in the size of the amplified product can indicate alternative splicing.

mRNA produced from mutant VEGF, Flt-1 or Flk-1/KDR genes can also be readily identified through the techniques described above for identifying alternative splice forms. As used herein, "mutant" VEGF gene or mRNA include human VEGF gene or mRNA which differ in sequence from the VEGF sequences set forth herein. Thus, allelic forms of these genes, and the mRNA produced from them, are considered "mutants" for purposes of this invention.

It is understood that human VEGF mRNA may contain target sequences in common with their respective alternative splice forms, cognates or mutants. A single siRNA comprising such a common targeting sequence can therefore induce RNAi-mediated degradation of different RNA types which contain the common targeting sequence.

The siRNA can comprise partially purified RNA, substantially pure RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, including modifications that make the siRNA resistant to nuclease digestion.

One or both strands of the siRNA can also comprise a 3' overhang. As used herein, a "3' overhang" refers to at least one unpaired nucleotide extending from the 3'-end of a duplexed RNA strand.

Thus in one embodiment, the siRNA comprises at least one 3' overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length.

In the embodiment in which both strands of the siRNA molecule comprise a 3' overhang, the length of the overhangs can be the same or different for each strand. In a most preferred embodiment, the 3' overhang is present on both strands of the siRNA, and is 2 nucleotides in length. For example, each strand of the siRNA can comprise 3' overhangs of dithymidylic acid ("TT") or diuridylic acid ("uu").

In order to enhance the stability of the present siRNA, the 3' overhangs can be also stabilized against degradation. In one embodiment, the overhangs are stabilized by including purine nucleotides, such as adenosine or guanosine nucleotides. Alternatively, substitution of pyrimidine nucleotides by modified analogues, *e.g.*, substitution of uridine nucleotides in the 3' overhangs with 2'-deoxythymidine, is tolerated and does not affect the efficiency of RNAi degradation. In particular, the absence of a 2' hydroxyl in the 2'-deoxythymidine significantly enhances the nuclease resistance of the 3'overhang in tissue culture medium.

In certain embodiments, the siRNA comprises the sequence AA(N19)TT or NA(N21), where N is any nucleotide. These siRNA comprise approximately 30-70% G/C content, and preferably comprise approximately 50% G/C content. The sequence of the sense siRNA strand corresponds to (N19)TT or N21 (i.e., positions 3 to 23), respectively. In the latter case, the 3' end of the sense siRNA is converted to TT. The rationale for this sequence conversion is to generate a symmetric duplex with respect to the sequence composition of the sense and antisense strand 3' overhangs. The antisense RNA strand is then synthesized as the complement to positions 1 to 21 of the sense strand.

Because position 1 of the 23-nt sense strand in these embodiments is not recognized in a sequence-specific manner by the antisense strand, the 3'-most nucleotide residue of the antisense strand can be chosen deliberately. However, the penultimate nucleotide of the antisense strand (complementary to position 2 of the 23-nt sense strand in either embodiment) is generally complementary to the targeted sequence.

In another embodiment, the siRNA comprises the sequence NAR(N17)YNN, where R is a purine (*e.g.*, A or G) and Y is a pyrimidine (*e.g.*, C or U/T). The respective 21-nt sense and antisense RNA strands of this embodiment therefore generally begin with a purine nucleotide. Such siRNA can be expressed from pol III expression vectors without a change in targeting site, as expression of RNAs from pol III promoters is only believed to be efficient when the first transcribed nucleotide is a purine.

In a further embodiment, the siRNA comprises a sequence having no more than five (5) consecutive purines or pyrimidines. In a further embodiment, the siRNA comprises a sequence having no more than five (5) consecutive nucleotides having the same nucleobase (i.e., A, C, G, or U/T).

The siRNA can be targeted to any stretch of approximately 19-25 contiguous nucleotides in any of the target mRNA sequences (the "target sequence"). Techniques for selecting target sequences for siRNA are given, for example, in Tuschl T et al., "The siRNA User Guide, " the entire disclosure of which is herein incorporated by reference. Thus, the sense strand of the present siRNA comprises a nucleotide sequence identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (*i.e.*, in the 3' direction) from the start codon. The target sequence can, however, be located in the 5' or 3' untranslated regions, or in the region nearby the start codon (see, *e.g.*, the target sequences of SEQ ID NOS: 73 and 74 in Table 1 below, which are within 100 nt of the 5'-end of the VEGF₁₂₁ cDNA.

In a further embodiment of the present invention, the target mRNA sequence comprises no more than five (5) consecutive purines or pyrimidines. For example, a suitable target sequence in the VEGF₁₂₁ cDNA sequence is:
TCATCACGAAGTGGTGAAG (SEQ ID NO: 8)

Thus, an siRNA targeting this sequence, and which has 3' uu overhangs on each strand (overhangs shown in bold), is:
5'-ucaucacgaaguggugaag**uu**-3' (SEQ ID NO: 9)
3'-**uu**aguagugcuucaccacuuc-5' (SEQ ID NO: 10)

An siRNA targeting this same sequence, but having 3' TT overhangs on each strand (overhangs shown in bold) is:
5'-ucaucacgaaguggugaag**TT**-3' (SEQ ID NO: 11)
3'-**TT**aguagugcuucaccacuuc-5' (SEQ ID NO: 12)

Other VEGF₁₂₁ target sequences from which siRNA can be derived are given in Table 1. It is understood that all VEGF₁₂₁ target sequences listed in Table 1 are within that portion of the VEGF₁₂₁ alternative splice form which is common to all human VEGF alternative splice forms. Thus, the VEGF₁₂₁ target sequences in Table 1 can also target VEGF₁₆₅, VEGF₁₈₉ and VEGF₂₀₆ mRNA. Target sequences which target a specific VEGF isoform can also be readily identified. For example, a target sequence which targets VEGF₁₆₅ mRNA but not VEGF₁₂₁ mRNA is AACGTACTTGCAGATGTGACA (SEQ ID NO: 13).

**Table 1- VEGF Target Sequences**

| **target sequence** | **SEQ ID NO:** | | **target sequence** | **SEQ ID NO:** |
|---|---|---|---|---|
| cognate VEGF mRNA sequence | 1 | | GATAGAGCAAGACAAGAAA | 26 |
| Splice variant VEGF₁₂₁ sequence | 2 | | GACAAGAAAATCCCTGTGG | 27 |
| Splice variant VEGF₁₆₅ sequence | 3 | | GAAAATCCCTGTGGGCCTT | 28 |
| Splice variant VEGF₁₈₉ sequence | 4 | | AATCCCTGTGGGCCTTGCT | 29 |
| Splice variant VEGF₂₀₆ sequence | 5 | | TCCCTGTGGGCCTTGCTCA | 30 |
| TCATCACGAAGTGGTGAAG | 8 | | GCATTTGTTTGTACAAGAT | 31 |
| ucaucacgaaguggugaag**uu** | 9 | | GATCCGCAGACGTGTAAAT | 32 |
| **uu**aguagugcuucaccacuuc | 10 | | ATGTTCCTGCAAAAACACA | 33 |
| ucaucacgaaguggugaag**TT** | 11 | | TGTTCCTGCAAAAACACAG | 34 |
| **TT**aguagugcuucaccacuuc | 12 | | AAACACAGACTCGCGTTGC | 35 |
| AACGTACTTGCAGATGTGACA | 13 | | AACACAGACTCGCGTTGCA | 36 |
| GTTCATGGATGTCTATCAG | 14 | | ACACAGACTCGCGTTGCAA | 37 |
| TCGAGACCCTGGTGGACAT | 15 | | CACAGACTCGCGTTGCAAG | 38 |
| TGACGAGGGCCTGGAGTGT | 16 | | GGCGAGGCAGCTTGAGTTA | 39 |
| TGACGAGGGCCTGGAGTGT | 17 | | ACGAACGTACTTGCAGATG | 40 |
| CATCACCATGCAGATTATG | 18 | | CGAACGTACTTGCAGATGT | 41 |
| ACCTCACCAAGGCCAGCAC | 19 | | CGTACTTGCAGATGTGACA | 42 |
| GGCCAGCACATAGGAGAGA | 20 | | GTGGTCCCAGGCTGCACCC | 43 |
| CAAATGTGAATGCAGACCA | 21 | | GGAGGAGGGCAGAATCATC | 44 |
| ATGTGAATGCAGACCAAAG | 22 | | GTGGTGAAGTTCATGGATG | 45 |
| TGCAGACCAAAGAAAGATA | 23 | | AATCATCACGAAGTGGTGAAG | 46 |
| AGAAAGATAGAGCAAGACA | 24 | | AAGTTCATGGATGTCTATCAG | 47 |
| GAAAGATAGAGCAAGACAA | 25 | | AATCGAGACCCTGGTGGACAT | 48 |
| AATGACGAGGGCCTGGAGTGT | 49 | | AATGTTCCTGCAAAAACACAGAC | 65 |
| AACATCACCATGCAGATTATG | 50 | | AAAAACACAGACTCGCGTTGCAA | 66 |
| AAACCTCACCAAGGCCAGCAC | 51 | | AAAACACAGACTCGCGTTGCAAG | 67 |
| AAGGCCAGCACATAGGAGAGA | 52 | | AAACACAGACTCGCGTTGCAAGG | 68 |
| "AACAAATGTGAATGCAGACCA | 53 | | AACACAGACTCGCGTTGCAAGGC | 69 |
| AAATGTGAATGCAGACCAAAG | 54 | | AAGGCGAGGCAGCTTGAGTTAA A | 70 |
| AATGCAGACCAAAGAAAGATA | 55 | | AAACGAACGTACTTGCAGATGTG | 71 |
| AAAGAAAGATAGAGCAAGACA | 56 | | AACGAACGTACTTGCAGATGTGA | 72 |
| AAGAAAGATAGAGCAACACAA | 57 | | AAGTGGTCCCAGGCTGCACCCAT | 73 |
| AAGATAGAGCAAGACAAGAAAA T | 58 | | AAGGAGGAGGGCAGAATCATCA C | 74 |
| AAGACAAGAAAATCCCTGTGGGC | 59 | | AAGTGGTGAAGTTCATGGATGTC | 75 |
| AAGAAAATCCCTGTGGGCCTTGC | 60 | | AAAATCCCTGTGGGCCTTGCTCA | 76 |
| AATCCCTGTGGGCCTTGCTCAGA | 61 | | accucaccaaggccagcacTT | 77 |
| AAGCATTTGTTTGTACAAGATCC | 62 | | gugcuggccuuggugagguTT | 78 |
| AAGATCCGCAGACGTGTAAATGT | 63 | | GGCTACGTCCAGCGCACC | 79 |
| AAATGTTCCTGCAAAAACACAGA | 64 | | AAACCUCACCAAAGCCAGCAC | 80 |

One embodiment of the present invention provides a method of stabilizing visual acuity comprising the administration of an effective amount of an siRNA targeting VEGF. In one particular embodiment of the invention, siRNA targeting VEGF is administered in an effective amount such that the subjects' visual acuity does not substantially decrease over time. In a preferred embodiment, siRNA comprising a sense RNA strand of SEQ ID NO: 77 and an antisense RNA strand of SEQ ID NO: 78 is administered in an effective amount such that the subjects' visual acuity does not substantially decrease over time.

In another embodiment of the invention, visual acuity is stabilized by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF-TRAP, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to stabilize visual acuity is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of stabilizing the visual acuity of a subject comprising administering an effective amount of an siRNA targeting VEGF wherein the effective amount of siRNA is administered weekly. Another embodiment of the invention comprises a method of stabilizing the visual acuity of a subject comprising administering an effective amount of an siRNA targeting VEGF wherein the effective amount of siRNA is administered every two weeks. Preferably, a further embodiment of the invention comprises a method of stabilizing the visual acuity of a subject by administering an effective amount of an siRNA targeting VEGF wherein the effective amount of siRNA is administered every four weeks. Additional preferred embodiments of the invention comprise methods of stabilizing the visual acuity of a subject by administering an effective amount of an siRNA targeting VEGF wherein the effective amount of siRNA is administered every eight weeks, twelve weeks, sixteen weeks or twenty weeks.

Another embodiment of the present invention provides a method of inhibiting choroidal neovascularization in a subject comprising the administration of an effective amount of an siRNA targeting VEGF. In a preferred embodiment, the siRNA, comprises a sense RNA strand of SEQ ID NO: 77 and an antisense RNA strand of SEQ ID NO: 78 is administered to a subject in an effective amount such that the subjects' choroidal neovascularization is inhibited.

In another embodiment of the invention, choroidal neovascularization is inhibited by administering to a subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF-TRAP, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to inhibit choroidal neovascularization is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of inhibiting choroidal neovascularization of a subject by administering an effective amount of an siRNA targeting VEGF, wherein the effective amount of siRNA is administered weekly. Another embodiment of the invention comprises a method of inhibiting choroidal neovascularization of a subject by administering an effective amount of an siRNA targeting VEGF, wherein the effective amount of siRNA is administered every two weeks. Preferably, a further embodiment of the invention comprises a method of inhibiting choroidal neovascularization of a subject by administering an effective amount of an siRNA targeting VEGF wherein the effective amount of siRNA is administered every four weeks. Additional preferred embodiments of the invention comprise methods of inhibiting choroidal neovascularization of a subject by administering an effective amount of an siRNA targeting VEGF wherein the effective amount of siRNA is administered every eight weeks, twelve weeks, sixteen weeks or twenty weeks.

One embodiment of the present invention provides a method of treating diabetic macular edema comprising the administration of an effective amount of an siRNA targeting VEGF. In a preferred embodiment, siRNA comprising a sense RNA strand of SEQ ID NO: 77 and an antisense RNA strand of SEQ ID NO: 78 is administered to a subject in an effective amount to treat the subjects' diabetic macular edema.

In another embodiment of the invention, diabetic macular edema may be treated by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF trap, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to treat diabetic macular edema is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of treating diabetic macular edema in a subject by administering an effective amount of an siRNA targeting VEGF wherein the effective amount of siRNA is administered weekly. Another embodiment of the invention comprises a method of treating diabetic macular edema in a subject by administering an effective amount of an siRNA targeting VEGF wherein the effective amount of siRNA is administered every two weeks. Preferably, a further embodiment of the invention comprises a method of treating diabetic macular edema of a subject by administering an effective amount of an siRNA targeting VEGF wherein the effective amount of siRNA is administered every four weeks. Additional preferred embodiments of the invention comprise methods of treating diabetic macular edema of a subject by administering an effective amount of an siRNA targeting VEGF wherein the effective amount of siRNA is administered every eight weeks, twelve weeks, sixteen weeks or twenty weeks.

One embodiment of the present invention provides a method of treating diabetic retinopathy comprising the administration of an effective amount of an siRNA targeting VEGF. In a preferred embodiment, siRNA comprising a sense RNA strand of SEQ ID NO: 77 and an antisense RNA strand of SEQ ID NO: 78 is administered to a subject in an effective amount to treat the subjects' diabetic retinopathy.

In another embodiment of the invention, diabetic retinopathy may be treated by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF trap, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to treat diabetic retinopathy is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of treating diabetic retinopathy in a subject by administering an effective amount of an siRNA targeting VEGF wherein the effective amount of siRNA is administered weekly. Another embodiment of the invention comprises a method of treating diabetic retinopathy in a subject by administering an effective amount of an siRNA targeting VEGF wherein the effective amount of siRNA is administered every two weeks. Preferably, a further embodiment of the invention comprises a method of treating diabetic retinopathy of a subject by administering an effective amount of an siRNA targeting VEGF wherein the effective amount of siRNA is administered every four weeks. Additional preferred embodiments of the invention comprise methods of treating diabetic retinopathy of a subject by administering an effective amount of an siRNA targeting VEGF wherein the effective amount of siRNA is administered every eight weeks, twelve weeks, sixteen weeks or twenty weeks.

One embodiment of the present invention provides a method of treating proliferative diabetic retinopathy comprising the administration of an effective amount of an siRNA targeting VEGF. In a preferred embodiment, siRNA comprising a sense RNA strand of SEQ ID NO: 77 and an antisense RNA strand of SEQ ID NO: 78 is administered to a subject in an effective amount to treat the subjects' proliferative diabetic retinopathy.

In another embodiment of the invention, proliferative diabetic retinopathy may be treated by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF trap, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to treat proliferative diabetic retinopathy is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of treating proliferative diabetic retinopathy in a subject by administering an effective amount of an siRNA targeting VEGF wherein the effective amount of siRNA is administered weekly. Another embodiment of the invention comprises a method of treating proliferative diabetic retinopathy in a subject by administering an effective amount of an siRNA targeting VEGF wherein the effective amount of siRNA is administered every two weeks. Preferably, a further embodiment of the invention comprises a method of treating proliferative diabetic retinopathy of a subject by administering an effective amount of an siRNA targeting VEGF wherein the effective amount of siRNA is administered every four weeks. Additional preferred embodiments of the invention comprise methods of treating proliferative diabetic retinopathy of a subject by administering an effective amount of an siRNA targeting VEGF wherein the effective amount of siRNA is administered every eight weeks, twelve weeks, sixteen weeks or twenty weeks.

One embodiment of the present invention provides a method of decreasing foveal thickness in a subject comprising the administration of an effective amount of an siRNA targeting VEGF. In one particular embodiment of the invention, siRNA targeting VEGF is administered to a subject in an effective amount such that the subjects' foveal thickness decreases over time. In a preferred embodiment, siRNA comprising a sense RNA strand of SEQ ID NO: 77 and an antisense RNA strand of SEQ ID NO: 78 is administered to a subject in an effective amount such that the subjects' foveal thickness decreases.

In another embodiment of the invention, the foveal thickness of a subject diagnosed with diabetic macular edema is decreased by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF trap, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to decrease foveal thickness is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of decreasing the foveal thickness of a subject by administering an effective amount of an siRNA targeting VEGF wherein the effective amount of siRNA is administered weekly. Another embodiment of the invention comprises a method of decreasing the foveal thickness of a subject by administering an effective amount of an siRNA targeting VEGF wherein the effective amount of siRNA is administered every two weeks. Preferably, a further embodiment of the invention comprises a method of decreasing the foveal thickness of a subject by administering an effective amount of an siRNA targeting VEGF wherein the effective amount of siRNA is administered every four weeks. Additional preferred embodiments of the invention comprise methods of decreasing the foveal thickness of a subject by administering an effective amount of an siRNA targeting VEGF wherein the effective amount of siRNA is administered every eight weeks, twelve weeks, sixteen weeks or twenty weeks.

One embodiment of the present invention provides a method of treating age-related macular degeneration in a subject comprising the administration of an effective amount of an siRNA targeting VEGF. In one particular embodiment of the invention, siRNA targeting VEGF is administered to a subject in an effective amount to treat the subjects' age-related macular degeneration. In a preferred embodiment siRNA comprising a sense RNA strand of SEQ ID NO: 77 and an antisense RNA strand of SEQ ID NO: 78 is administered to a subject in an effective amount to treat the subjects' age-related macular degeneration.

In another embodiment of the invention, age-related macular degeneration is treated by administering to the subject an siRNA targeting VEGF and administering a pharmaceutical agent that is different from the siRNA, preferably a VEGF antagonist. VEGF antagonists comprising a number of distinct pharmaceutical classes have been developed. In one embodiment, the VEGF antagonist comprises a monoclonal antibody that targets the VEGF protein, such as bevacizumab or ranibizumab. In another embodiment, the VEGF antagonist is a VEGF trap, such as aflibercept. In yet another embodiment, the VEGF antagonist is an aptamer, such as pegaptanib. In a preferred embodiment, the siRNA administered with the VEGF antagonist comprises a sense RNA strand of SEQ ID NO: 77 and an antisense RNA strand of SEQ ID NO: 78.

In an embodiment of the invention, the effective amount of the siRNA targeting VEGF to treat age-related macular degeneration is from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. In a preferred embodiment, the siRNA targeting VEGF administered to a subject comprises a sense RNA strand of SEQ ID NO: 77 and an antisense RNA strand of SEQ ID NO: 78.

An embodiment of the invention comprises a method of treating age-related macular degeneration in a subject by administering an effective amount of an siRNA targeting VEGF wherein the effective amount of siRNA is administered weekly. Another embodiment of the invention comprises a method of treating age-related macular degeneration in a subject by administering an effective amount of an siRNA targeting VEGF wherein the effective amount of siRNA is administered every two weeks. Preferably, a further embodiment of the invention comprises a method of treating age-related macular degeneration of a subject by administering an effective amount of an siRNA targeting VEGF wherein the effective amount of siRNA is administered every four weeks. Additional preferred embodiments of the invention comprise methods of treating age-related macular degeneration of a subject by administering an effective amount of an siRNA targeting VEGF wherein the effective amount of siRNA is administered every eight weeks, twelve weeks, sixteen weeks or twenty weeks.

Other embodiments of the present invention provide methods of treating age-related macular degeneration and diabetic macular edema comprising administering to a subject an effective amount of a VEGF antagonist and an effective amount of an siRNA comprising a sense RNA strand and an antisense RNA strand, wherein the sense and the antisense RNA strands form an RNA duplex, and wherein the sense RNA strand comprises a nucleotide sequence identical to a target sequence of about 19 to about 25 contiguous nucleotides in human VEGF mRNA. The VEGF antagonist may be administered prior to, after or simultaneously with the siRNA. In preferred embodiments, the sense RNA strand comprises SEQ ID NO: 77 and the antisense strand comprises SEQ ID NO: 78. The siRNA may be administered by intraocular administration, such as intravitreal, intraretinal, subretinal, subtenon, peri- and retro-orbital, trans-corneal and trans-scleral administration. In such methods, the effective amount of said siRNA may be from about 0.1 mg to about 20 mg of siRNA. In a further embodiment, the effective amount is from about 0.2 mg to about 10 mg of siRNA targeting VEGF. In an additional embodiment, preferably the effective amount of the siRNA is from about 0.5 to about 5 mg of siRNA targeting VEGF. Further preferred embodiments provide effective amounts of about 1 mg to about 3 mg, including about 1.5 mg, 2.5 mg or about 3 mg of siRNA targeting VEGF. The effective amount of said VEGF siRNA may be administered weekly, every two weeks, every four weeks, every eight weeks, every twelve weeks, every sixteen weeks, or every twenty weeks. Preferably, the VEGF siRNA is administered every four weeks.

In certain embodiments, the VEGF antagonist is a monoclonal antibody targeting human VEGF, such as, for example, bevacizumab or ranibizumab. In other embodiments, the VEGF antagonist is a VEGF trap, such as aflibercept. In other embodiments, the VEGF antagonist is a VEGF aptamer, such as pegaptanib.

In one embodiment, such a method comprises administering a VEGF siRNA, preferably the sense RNA strand comprises SEQ ID NO: 77 and the antisense strand comprises SEQ ID NO: 78, and ranibizumab. The siRNA may be administered every four weeks (i.e. monthly) and said ranibizumab may be administered every four weeks (monthly). In a further embodiment, the ranibizumab may be administered two weeks prior to administration of said siRNA on an alternating basis. For example, one embodiment may comprise administering an effective amount of ranibizumab on day 0, then administering an effective amount of VEGF siRNA week 2, then administering an effective amount of ranibizumab week four, then administering an effective amount of VEGF siRNA week six, then administering an effective amount of ranibizumab week eight. Such an administration may comprise an initiation therapy to treat the age-related macular degeneration. In a further embodiment, the initiation therapy may be followed by a maintenance therapy, wherein starting at week 12 the siRNA is administered, for example, every eight weeks or every twelve weeks.

In one embodiment, such a method comprises administering a VEGF siRNA, preferably the sense RNA strand comprises SEQ ID NO: 77 and the antisense strand comprises SEQ ID NO: 78, and bevacizumab. The siRNA may be administered every four weeks (i.e. monthly) and said bevacizumab may be administered every four weeks (monthly). In a further embodiment, the bevacizumab may be administered two weeks prior to administration of said siRNA on an alternating basis. For example, one embodiment may comprise administering an effective amount of bevacizumab on day 0, then administering an effective amount of VEGF siRNA week 2, then administering an effective amount of bevacizumab week four, then administering an effective amount of VEGF siRNA week six, then administering an effective amount of bevacizumab week eight. Such an administration may comprise an initiation therapy to treat the age-related macular degeneration. In a further embodiment, the initiation therapy may be followed by a maintenance therapy, wherein starting at week 12 the siRNA is administered, for example, every eight weeks or every twelve weeks.

In another embodiment, such a method comprises administering a VEGF siRNA, preferably the sense RNA strand comprises SEQ ID NO: 77 and the antisense strand, comprises SEQ ID NO: 78, and a VEGF antagonist, such as, for example, ranibizumab, bevacizumab, aflibercept or pegaptanib. The siRNA may be administered every four weeks (i.e. monthly) and said VEGF antagonist may be administered every four weeks (monthly). In a further embodiment, the VEGF antagonist may be administered two weeks prior to administration of said siRNA on an alternating basis. For example, one embodiment may comprise administering an effective amount of VEGF antagonist on day 0, then administering an effective amount of VEGF siRNA week 2, then administering an effective amount of VEGF antagonist week four, then administering an effective amount of VEGF siRNA week six, then administering an effective amount of VEGF antagonist week eight. Such an administration may comprise an initiation therapy to treat the age-related macula degeneration. In a further embodiment, the initiation therapy may be followed by a maintenance therapy, wherein starting at week 12 the siRNA is administered, for example, every eight weeks or every twelve weeks.

Embodiments of the present invention also include pharmaceutical compositions comprising an siRNA comprising a sense RNA strand of SEQ ID NO: 77 and an antisense RNA strand of SEQ ID NO: 78, wherein the siRNA, is in a balanced salt solution. Another embodiment of the invention is a pharmaceutical composition comprising an siRNA comprising a sense RNA strand of SEQ ID NO: 77 and an antisense RNA strand of SEQ ID NO: 78 and a balanced salt solution such that the pharmaceutical composition is suitable for ocular administration. An additional embodiment of the invention is a pharmaceutical composition comprising an siRNA comprising a sense RNA strand of SEQ ID NO: 77 and an antisense RNA strand of SEQ ID NO: 78 and a balanced salt solution wherein the pharmaceutical composition has a pH of about 6.08 to about 8.0. Further embodiments include pharmaceutical compositions comprising an siRNA comprising a sense RNA strand of SEQ ID NO: 77 and an antisense RNA strand of SEQ ID NO: 78 wherein the siRNA is at a concentration of 0.2 mg/100 µL to about 3.0 mg/100 µL. Additional embodiments of the invention are pharmaceutical compositions comprising an siRNA comprising a sense RNA strand of SEQ ID NO: 77 and an antisense RNA strand of SEQ ID NO: 78 wherein the siRNA is at a concentration of 1.5 mg/100 µL, 2.5 mg/100 µL or 3.0 mg/100 µL. Further embodiments include pharmaceutical compositions comprising an siRNA comprising a sense RNA strand of SEQ ID NO: 77 and an antisense RNA strand of SEQ ID NO: 78 wherein the siRNA is at a concentration of 0.2 mg/50 µL to about 3.0 mg/50 µL. Additional embodiments of the invention are pharmaceutical compositions comprising an siRNA comprising a sense RNA strand of SEQ ID NO: 77 and an antisense RNA strand of SEQ ID NO: 78 wherein the siRNA is at a concentration of 1.5 mg/50 µL, 2.5 mg/50 µL or 3.0 mg/50 µL.

Another embodiment is a pharmaceutical composition suitable for ocular administration comprising an siRNA comprising a sense RNA strand of SEQ ID NO: 77 and an antisense RNA strand of SEQ ID NO: 78, sodium acetate (trihydrate), sodium chloride, sodium citrate(tribasic dehydrate) potassium chloride, calidum chloride (dehydrate), magnesium chloride (hexahydrate), hydrochloric acid, sodium hydroxide and water.

The siRNA can be obtained using a number of techniques known to those of skill in the art. For example, the siRNA can be chemically synthesized or recombinantly produced using methods known in the art, such as the Drosophila *in vitro* system described in U.S. published application 2002/0086356 of Tuschl et al., the entire disclosure of which is herein incorporated by reference.

Preferably, the siRNA are chemically synthesized using appropriately protected ribonucleoside phosphoramidites and a conventional DNA/RNA synthesizer. The siRNA can be synthesized as two separate, complementary RNA molecules, or as a single RNA molecule with two complementary regions. Commercial suppliers of synthetic RNA molecules or synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA) and Cruachem (Glasgow, UK). The siRNA can also be synthesized as multiple complementary RNA molecules, as described in more detail in co-pending U.S. Provisional Application No. 60/824,953, entitled "siRNA and Methods of Manufacture" filed September 8, 2006, herein incorporated by reference in its entirety.

Alternatively, siRNA can also be expressed from recombinant circular or linear DNA plasmids using any suitable promoter. Suitable promoters for expressing siRNA from a plasmid include, for example, the U6 or H1 RNA pol III promoter sequences and the cytomegalovirus promoter. Selection of other suitable promoters is within the skill in the art. The recombinant plasmids of the invention can also comprise inducible or regulatable promoters for expression of the siRNA in a particular tissue or in a particular intracellular environment.

The siRNA expressed from recombinant plasmids can either be isolated from cultured cell expression systems by standard techniques, or can be expressed intracellularly at or near the area of neovascularization *in vivo.* The use of recombinant plasmids to deliver siRNA to cells *in vivo* is discussed in more detail below.

siRNA can be expressed from a recombinant plasmid either as two separate, complementary RNA molecules, or as a single RNA molecule with two complementary regions.

Selection of plasmids suitable for expressing siRNA, methods for inserting nucleic acid sequences for expressing the siRNA into the plasmid, and methods of delivering the recombinant plasmid to the cells of interest are within the skill in the art. See, for example Tuschl, T. (2002), Nat. Biotechnol, 20: 446-448; Brummelkamp TR et al. (2002), Science 296: 550-553; Miyagishi M et al. (2002), Nat. Biotechnol. 20: 497-500; Paddison PJ et al. (2002), Genes Dev. 16: 948-958; Lee NS et al. (2002), Nat. Biotechnol. 20: 500-505; and Paul CP et al. (2002), Nat. Biotechnol. 20: 505-508, the entire disclosures of which are herein incorporated by reference.

A plasmid comprising nucleic acid sequences for expressing an siRNA is described in Example 7 below. That plasmid, called pAAVsiRNA, comprises a sense RNA strand coding sequence in operable connection with a polyT termination sequence under the control of a human U6 RNA promoter, and an antisense RNA strand coding sequence in operable connection with a polyT termination sequence under the control of a human U6 RNA promoter. The plasmid pAAVsiRNA is ultimately intended for use in producing a recombinant adeno-associated viral vector comprising the same nucleic acid sequences for expressing an siRNA.

As used herein, "in operable connection with a polyT termination sequence" means that the nucleic acid sequences encoding the sense or antisense strands are immediately adjacent to the polyT termination signal in the 5' direction. During transcription of the sense or antisense sequences from the plasmid, the polyT termination signals act to terminate transcription.

As used herein, "under the control" of a promoter means that the nucleic acid sequences encoding the sense or antisense strands are located 3' of the promoter, so that the promoter can initiate transcription of the sense or antisense coding sequences.

The siRNA can also be expressed from recombinant viral vectors intracellularly at or near the area of neovascularization *in vivo.* The recombinant viral vectors of the invention comprise sequences encoding the siRNA and any suitable promoter for expressing the siRNA sequences. Suitable promoters include, for example, the U6 or H1 RNA pol III promoter sequences and the cytomegalovirus promoter. Selection of other suitable promoters is within the skill in the art. The recombinant viral vectors of the invention can also comprise inducible or regulatable promoters for expression of the siRNA in a particular tissue or in a particular intracellular environment. The use of recombinant viral vectors to deliver siRNA to cells *in vivo* is discussed in more detail below.

siRNA can be expressed from a recombinant viral vector either as two separate, complementary RNA molecules, or as a single RNA molecule with two complementary regions.

Any viral vector capable of accepting the coding sequences for the siRNA molecule(s) to be expressed can be used, for example vectors derived from adenovirus (AV); adeno-associated virus (AAV); retroviruses (*e.g*, lentiviruses (LV), Rhabdoviruses, murine leukemia virus); herpes virus, and the like. The tropism of the viral vectors can also be modified by pseudotyping the vectors with envelope proteins or other surface antigens from other viruses. For example, an AAV vector of the invention can be pseudotyped with surface proteins from vesicular stomatitis virus (VSV), rabies, Ebola, Mokola, and the like.

Selection of recombinant viral vectors suitable for use in the invention, methods for inserting nucleic acid sequences for expressing the siRNA into the vector, and methods of delivering the viral vector to the cells of interest are within the skill in the art. See, for example, Dornburg R (1995), Gene Therap. 2: 301-310; Eglitis MA (1988), Biotechniques 6: 608-614; Miller AD (1990), Hum Gene Therap. 1: 5-14; and Anderson WF (1998), Nature 392: 25-30, the entire disclosures of which are herein incorporated by reference.

Preferred viral vectors are those derived from AV and AAV. In a particularly preferred embodiment, the siRNA is expressed as two separate, complementary single-stranded RNA molecules from a recombinant AAV vector comprising, for example, either the U6 or H1 RNA promoters, or the cytomegalovirus (CMV) promoter.

A suitable AV vector for expressing the siRNA, a method for constructing the recombinant AV vector, and a method for delivering the vector into target cells, have been described.

Suitable AAV vectors for expressing the siRNA, methods for constructing the recombinant AAV vector, and methods for delivering the vectors into target cells are described in Samulski R et al. (1987), J Virol. 61: 3096-3101; Fisher KJ et al. (1996), J. Virol., 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641, the entire disclosures of which are herein incorporated by reference.

As discussed above, the siRNA is capable of targeting and causing the RNAi-mediated degradation of VEGF, more preferably human VEGF. Degradation of the target mRNA by the present siRNA reduces the production of a functional gene product from the VEGF. Thus, another embodiment of the present invention provides a method of inhibiting expression of VEGF in a subject, comprising administering an effective amount of an siRNA to the subject, such that the target mRNA is degraded. As the products of the VEGF gene is required for initiating and maintaining angiogenesis, another embodiment of the present invention provides a method of inhibiting angiogenesis in a subject by the RNAi-mediated degradation of the target mRNA by the present siRNA.

RNAi-mediated degradation of the target mRNA can be detected by measuring levels of the target mRNA or protein in the cells of a subject, using standard techniques for isolating and quantifying mRNA or protein as described above.

Inhibition of angiogenesis can be evaluated by directly measuring the progress of pathogenic or nonpathogenic angiogenesis in a subject; for example, by observing the size of a neovascularized area before and after treatment with the siRNA. An inhibition of angiogenesis is indicated if the size of the neovascularized area stays the same or is reduced. Techniques for observing and measuring the size of neovascularized areas in a subject are within the skill in the art; for example, areas of choroid neovascularization can be observed by ophthalmoscopy.

Inhibition of angiogenesis can also be inferred through observing a change or reversal in a pathogenic condition associated with the angiogenesis. For example, in ARMD, a slowing, halting or reversal of vision loss indicates an inhibition of angiogenesis in the choroid.

It is understood that the siRNA can degrade the target mRNA (and thus inhibit angiogenesis) in substoichiometric amounts. Without wishing to be bound by any theory, it is believed that the siRNA causes degradation of the target mRNA in a catalytic manner. Thus, compared to standard anti-angiogenic therapies, significantly less siRNA needs to be delivered at or near the site of neovascularization to have a therapeutic effect.

In certain embodiments, an effective amount of siRNA is from about 0.1 mg to about 20 mg. In other embodiments, an effective amount of siRNA is from about 0.2 mg to about 20 mg. In preferred embodiments, an effective amount of siRNA is from about 0.5 mg to about 5 mg, more preferably about 1 mg to about 3 mg, including about 1.5 mg, about 2.5 mgs and about 3.0 mg.

Other angiogenic diseases include diabetic retinopathy, age-related macular degeneration (ARMD), psoriasis, rheumatoid arthritis and other inflammatory diseases. These diseases are characterized by the destruction of normal tissue by newly formed blood vessels in the area of neovascularization. For example, in ARMD, the choroid is invaded and destroyed by capillaries. The angiogenesis-driven destruction of the choroid in ARMD eventually leads to partial or full blindness.

More preferably, an siRNA is used to inhibit choroidal neovascularization in age-related macular degeneration.

For treating angiogenic diseases, the siRNAs can be administered to a subject in combination with a pharmaceutical agent which is different from the present siRNA. Alternatively, the siRNA can be administered to a subject in combination with another therapeutic method designed to treat the angiogenic disease. For example, the siRNA can be administered in combination with therapeutic methods currently employed for treating age-related macular degeneration.

In the present methods, the present siRNA can be administered to the subject either as naked siRNA, in conjunction with a delivery reagent, or as a recombinant plasmid or viral vector which expresses the siRNA.

Suitable delivery reagents for administration in conjunction with the present siRNA include the Mirus Transit TKO lipophilic reagent; lipofectin; lipofectamine; cellfectin; or polycations (e.g., polylysine), or liposomes. A preferred delivery reagent is a liposomes.

Liposomes can aid in the delivery of the siRNA to a particular tissue, such as retinal or tumor tissue, and can also increase the blood half-life of the siRNA. Liposomes suitable for use in the invention are formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example as described in Szoka et al. (1980), Ann. Rev. Biaphys. Bioeng. 9: 467; and U.S. Pat. Nos. 4,235,871, 4,501,728, 4,837,028, and 5,019,369, the entire disclosures of which are herein incorporated by reference.

Preferably, the liposomes encapsulating the present siRNA comprises a ligand molecule that can target the liposome to a particular cell or tissue at or near the site of angiogenesis. Ligands which bind to receptors prevalent in tumor or vascular endothelial cells, such as monoclonal antibodies that bind to tumor antigens or endothelial cell surface antigens, are preferred.

Particularly preferably, the liposomes encapsulating the present siRNA are modified so as to avoid clearance by the mononuclear macrophage and reticuloendothelial systems, for example by having opsonization-inhibition moieties bound to the surface of the structure. In one embodiment, a liposome of the invention can comprise both opsonization-inhibition moieties and a ligand.

Opsonization-inhibiting moieties for use in preparing the liposomes of the invention are typically large hydrophilic polymers that are bound to the liposome membrane. As used herein, an opsonization inhibiting moiety is "bound" to a liposome membrane when it is chemically or physically attached to the membrane, *e.g.*, by the intercalation of a lipid-soluble anchor into the membrane itself, or by binding directly to active groups of membrane lipids. These opsonization-inhibiting hydrophilic polymers form a protective surface layer which significantly decreases the uptake of the liposomes by the macrophage-monocyte system ("MMS") and reticuloendothelial system ("RES"); *e.g*., as described in U.S. Pat. No. 4,920,016, the entire disclosure of which is herein incorporated by reference. Liposomes modified with opsonization-inhibition moieties thus remain in the circulation much longer than unmodified liposomes. For this reason, such liposomes are sometimes called "stealth" liposomes.

Stealth liposomes are known to accumulate in tissues fed by porous or "leaky" microvasculature. Thus, target tissue characterized by such microvasculature defects, for example solid tumors, will efficiently accumulate these liposomes. In addition, the reduced uptake by the RES lowers the toxicity of stealth liposomes by preventing significant accumulation in the liver and spleen. Thus, liposomes of the invention that are modified with opsonization-inhibition moieties can deliver the present siRNA to tumor cells.

Opsonization inhibiting moieties suitable for modifying liposomes are preferably water-soluble polymers with a number-average molecular weight from about 500 to about 40,000 daltons, and more preferably from about 2,000 to about 20,000 daltons. Such polymers include polyethylene glycol (PEG) or polypropylene glycol (PPG) derivatives; *e.g*., methoxy PEG or PPG, and PEG or PPG stearate; synthetic polymers such as polyacrylamide or poly N-vinyl pyrrolidone; linear, branched, or dendrimeric polyarnidoamines; polyacrylic acids; polyalcohols, e.g., polyvinylalcohol and polyxylitol to which carboxylic or amino groups are chemically linked, as well as gangliosides, such as ganglioside GM₁. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, e.g., reacted with derivatives of carbonic acids with resultant linking of carboxylic groups.

Preferably, the opsonization-inhibiting moiety is a PEG, PPG, or derivatives thereof. Liposomes modified with PEG or PEG-derivatives are sometimes called "PEGylated liposomes."

The opsonization inhibiting moiety can be bound to the liposome membrane by any one of numerous well-known techniques. For example, an N-hydroxysuccinimide ester of PEG can be bound to a phosphatidyl-ethanolamine lipid-soluble anchor, and then bound to a membrane. Similarly, a dextran polymer can be derivatized with a stearylamine lipid-soluble anchor via reductive amination using Na(CN)BH₃ and a solvent mixture such as tetrahydrofuran and water in a 30:12 ratio at 60 °C.

Recombinant plasmids which express siRNA are discussed above. Such recombinant plasmids can also be administered directly or in conjunction with a suitable delivery reagent, including the Mirus Transit LT1 lipophilic reagent; lipofectin; lipofectamine; cellfectin; polycations (*e.g*., polylysine) or liposomes. Recombinant viral vectors which express siRNA are also discussed above, and methods for delivering such vectors to an area of neovascularization in a patient are within the skill in the art.

The siRNA can be administered to the subject by any means suitable for delivering the siRNA to the cells of the tissue at or near the area of neovascularization. For example, the siRNA can be administered by gene gun, electroporation, or by other suitable parenteral or enteral administration routes.

Suitable enteral administration routes include oral, rectal, or intranasal delivery.

Suitable parenteral administration routes include intravascular administration (e.g. intravenous bolus injection, intravenous infusion, intra-arterial bolus injection, intra-arterial infusion and catheter instillation into the vasculature); peri- and intra-tissue administration (*e.g*., peri-tumoral and intra-tumoral injection, intra-retinal injection, subretinal injection or intravitreal injection); subcutaneous injection or deposition including subcutaneous infusion (such as by osmotic pumps); direct (*e.g.*, topical) application to the area at or near the site of neovascularization, for example by a catheter or other placement device (*e.g*., a corneal pellet or a suppository, eye-dropper, or an implant comprising a porous, non-porous, or gelatinous material); and inhalation. Suitable placement devices include the ocular implants described in U.S. Pat. Nos. 5,902,598 and 6,375,972, and the biodegradable ocular implants described in U.S. Pat. No 6,331,313, the entire disclosures of which are herein incorporated by reference. Such ocular implants are available from Control Delivery Systems, Inc. (Watertown, MA) and Oculex Pharmaceuticals, lnc. (Sunnyvale, CA).

In a preferred embodiment, injections or infusions of the siRNA are given at or near the site of neovascularization. More preferably, the siRNA is administered topically to the eye, *e.g.* in liquid or gel form to the lower eye lid or conjunctival cul-de-sac, as is within the skill in the art (see, *e.g*., Acheampong AA et al, 2002, Drug Metabol. and Disposition 30: 421-429, the entire disclosure of which is herein incorporated by reference).

Typically, the siRNA is administered topically to the eye in amounts of from about 5 microliters to about 75 microliters, for example from about 7 microliters to about 50 microliters, preferably from about 10 microliters to about 30 microliters. It is understood that topical instillation in the eye of siRNA in volumes greater than 75 microliters can result in loss of siRNA from the eye through spillage and drainage.

A particularly preferred parenteral administration route is intraocular administration. It is understood that intraocular administration of the present siRNA can be accomplished by injection or direct (e.g., topical) administration to the eye, as long as the administration route allows the siRNA to enter the eye. In addition to the topical routes of administration to the eye described above, suitable intraocular routes of administration include intravitreal, intraretinal, subretinal, subtenon, peri- and retro-orbital, trans-corneal and trans-scleral administration. Such intraocular administration routes are within the skill in the art; see, *e.g*., and Acheampong AA et al, 2002, *supra*; and Bennett et al. (1996), Hum. Gene Ther. 7: 1763-1769 and Ambati J et al., 2002, Progress in Retinal and Eye Res. 21: 145-151, the entire disclosures of which are herein incorporated by reference. In another preferred embodiment, the siRNA is administered by intravitreal injection.

The siRNA can be administered in a single dose or in multiple doses. Where the administration of the siRNA is by infusion, the infusion can be a single sustained dose or can be delivered by multiple infusions. Injection of the agent directly into the tissue is at or near the site of neovascularization preferred. Multiple injections of the agent into the tissue at or near the site of neovascularization are particularly preferred.

The siRNA can be administered to the subject once, such as by a single injection or deposition at or near the neovascularization site. Alternatively, the siRNA can be administered to a subject multiple times daily or weekly. For example, the siRNA can be administered to a subject once weekly for a period of from about three to about twenty-eight weeks, more preferably from about seven to about ten weeks. In a preferred dosage regimen, the siRNA is injected at or near the site of neovascularization once every four weeks, eight weeks, or twelve weeks, In a more preferred dosage regimen, the siRNA is injected at or near the site of neovascularization (*e.g*., intravitreally) once every four weeks during an initiation period, preferably about eight weeks, followed by a maintenance period wherein said siRNA is injected once every eight to twelve weeks. Preferably, the dose of siRNA is preceded by administration of a VEGF antagonist, preferably two weeks prior to administration of the siRNA, and for a period of eight weeks (i.e.- the initiation period). It is understood that periodic administrations of the siRNA for an indefinite length of time may be necessary for subjects suffering from a chronic neovascularization disease, such as wet ARMD or diabetic retinopathy.

Where a dosage regimen comprises multiple administrations, it is understood that the effective amount of siRNA administered to the subject can comprise the total amount of siRNA administered over the entire dosage regimen.

The siRNA are preferably formulated as pharmaceutical compositions prior to administering to a subject, according to techniques known in the art. Pharmaceutical compositions of the present invention are characterized as being at least sterile and pyrogen-free. As used herein, "pharmaceutical formulations" include formulations for human and veterinary use. Methods for preparing pharmaceutical compositions of the invention are within the skill in the art, for example as described in Remington's Pharmaceutical Science, 17th ed., Mack Publishing Company, Easton, Pa. (1985), the entire disclosure of which is herein incorporated by reference.

In one embodiment, the pharmaceutical formulations comprise an siRNA (*e.g.,* 0.1 to 90% by weight), or a physiologically acceptable salt thereof, mixed with a physiologically acceptable carrier medium. Preferred physiologically acceptable carrier media are water, buffered water, saline solutions (*e.g*., normal saline or balanced saline solutions such as Hank's or Earle's balanced salt solutions), 0.4% saline, 0.3% glycine, hyaluronic acid and the like.

Pharmaceutical compositions can also comprise conventional pharmaceutical excipients and/or additives. Suitable pharmaceutical excipients include stabilizers, antioxidants, osmolality adjusting agents, buffers, and pH adjusting agents. Suitable additives include physiologically biocompatible buffers (*e.g*., tromethamine hydrochloride), additions of chelants (such as, for example, DTPA or DTPA-bisamide) or calcium chelate complexes (as for example calcium DTPA, CaNaDTPA-bisamide), or, optionally, additions of calcium or sodium salts (for example, calcium chloride, calcium ascorbate, calcium gluconate or calcium lactate). Pharmaceutical compositions of the invention can be packaged for use in liquid form, or can be lyophilized.

For topical administration to the eye, conventional intraocular delivery reagents can be used. For example, pharmaceutical compositions of the invention for topical intraocular delivery can comprise saline solutions as described above, corneal penetration enhancers, insoluble particles, petrolatum or other gel-based ointments, polymers which undergo a viscosity increase upon instillation in the eye, or mucoadhesive polymers. Preferably, the intraocular delivery reagent increases corneal penetration, or prolongs preocular retention of the siRNA through viscosity effects or by establishing physicochemical interactions with the mucin layer covering the corneal epithelium.

Suitable insoluble particles for topical intraocular delivery include the calcium phosphate particles described in U.S. Pat. No. 6,355,271 of Bell et al., the entire disclosure of which is herein incorporated by reference. Suitable polymers which undergo a viscosity increase upon instillation in the eye include polyethylenepolyoxypropylene block copolymers such as poloxamer 407 (*e.g*., at a concentration of 25%), cellulose acetophthalate (*e.g*., at a concentration of 30%), or a low-acetyl gellan gum such as Gelrite® (available from CP Kelco, Wilmington, DE). Suitable mucoadhesive polymers include hydrocolloids with multiple hydrophilic functional groups such as carboxyl, hydroxyl, amide and/or sulfate groups; for example, hydroxypropylcellulose, polyacrylic acid, high-molecular weight polyethylene glycols (*e.g*., >200,000 number average molecular weight), dextrans, hyaluronic acid, polygalacturonic acid, and xylocan. Suitable corneal penetration enhancers include cyclodextrins, benzalkonium chloride, polyoxyethylene glycol lauryl ether (*e.g*., Brij® 35), polyoxyethylene glycol stearyl ether (*e.g*., Brij® 78), polyoxyethylene glycol oleyl ether (*e.g*., Brij® 98), ethylene diamine tetraacetic acid (EDTA), digitonin, sodium taurocholate, saponins and polyoxyethylated castor oil such as Cremaphor EL.

For solid compositions, conventional nontoxic solid carriers can be used; for example, pharmaceutical grades of mannitol, lactose, starch, magnesium stearate, sodium saccharin, talcum, cellulose, glucose, sucrose, magnesium carbonate, and the like.

For example, a solid pharmaceutical composition for oral administration can comprise any of the carriers and excipients listed above and 10-95%, preferably 25%-75%, of one or more siRNA. A pharmaceutical composition for aerosol (inhalational) administration can comprise 0.01-20% by weight, preferably 1%-10% by weight, of one or more siRNA encapsulated in a liposome as described above, and propellant. A carrier can also be included as desired; e.g., lecithin for intranasal delivery.

The invention will now be illustrated with the following non-limiting examples.

**Example 1** - ***In Vivo* RNA Interference of VEGF in Monkevs with Anti-VEGF siRNA**

*siRNAs Design -* A 19 nt sequence located 329 nt from the 5' end of human VEGF mRNA was chosen as a target sequence: AAACCTCACCAAGGCCAGCAC (SEQ ID NO: 51). To ensure that it was not contained in the RNA from any other genes, this target sequence was entered into the BLAST search engine provided by NCBI. The use of the BLAST algorithm is described in Altschul et al. (1990), J. Mol. Biol. 215: 403-410 and Altschul et al. (1997), Nucleic Acids Res. 25: 3389-3402, the disclosures of which are herein incorporated by reference in their entirety. As no other mRNA was found which contained the target sequence, an siRNA duplex was synthesized to target this sequence (Dharmacon Research, Inc., Lafayette, CO).

The siRNA duplex had the following sense and antisense strands. sense:
5'-accucaccaaggccagcacTT-3' (SEQ ID NO: 77). antisense:
5'-gugcuggccuuggugagguTT-3' (SEQ ID NO: 78).

Together, the siRNA sense and antisense strands formed a 19 nt double-stranded siRNA with TT 3' overhangs (shown in bold) on each strand. This siRNA was termed "Candidate 5" or "can5." Other siRNA which target human VEGF mRNA were designed and tested as described for Cand5.

The objectives of this study were to determine the safety and efficacy of Cand5 when administered by single intravitreal injection to male cynomolgus monkeys following induction of CNV. Cand5 was administered in the vehicle control article to naive male cynomolgus monkeys in the following dose levels: 0 mg/eye (control), 0.07 mg/eye, 0.18 mg/eye, 0.35 mg/eye and, and 0.70 mg/eye.

CNV was induced by laser treatment to the maculae of both eyes of each animal, and the doses of Cand5 were given shortly following laser treatment. The animals were evaluated for changes in clinical signs, body weight and ocular condition (extensive ophthalmic examinations, electroretinography and tonometry). Fluorescein angiography was performed and blood samples were collected. At the end of the study (Day 44), all animals were euthanized and a complete gross necropsy was performed. Selected tissues were collected and preserved for histopathologic evaluation.

No adverse systemic or local (ocular) effects of Cand5 were detected when monkeys were administered a single intravitreal injection into both eyes at doses up to 0.70 mg/eye following laser lesioning of the macula and during subsequent development of CNV.

**Example 2 - *In Vitro* RNA Interference of VEGF' with Anti-VEGF siRNA in Human Embryonic Kidney 293 Cells**

Human embryonic kidney 293 cells (obtained from ATCC, Manassas, VA) were cultured in Dulbecco's Modified Eagle Medium (DMEM; obtained from Cellgro, Herndon, VA) with 10% fetal bovine serum (FBS; from JRH Biosciences, Lenexa, KS) and an antibiotic-antimycotic reagent, used for the prevention of cell culture growth contaminants (from Gibco, Carlsbad, CA).

siRNAs were synthesized by Integrated DNA Technologies (Coralville, IA). The siRNA target sequences are shown in Table 2. An additional siRNA was used in this study that targets the gene of enhanced green fluorescent protein (EGFP) as a negative control.

**Table 2.**

| **siRNAs Name** | **GC Content** | **Nucleotide Start Site** | **Target Sequence 5'-3' of siRNA** |
|---|---|---|---|
| HVEGF#1 | 58% | 92 | aaggaggagggeagaateatc (SEQ ID NO: 81) |
| hVEGF#2 | 42% | 124 | aagttcatggatgtctatcag (SEQ ID NO: 47) |
| hVEGF#3 | 58% | 162 | aatcgagaccctggtggacat (SEQ ID NO: 48) |
| hVEGF#4 | 42% | 301 | aacatcaccatgcagattatg (SEQ ID NO: 50) |
| hVEGF#5 | 58% | 338 | aaggccagcacataggagaga (SEQ ID NO: 52) |
| hVEGF#6 | 42% | 380 | aatgtgaatgcagaccaaaga (SEQ ID NO: 82) |
| hVEGF#7 | 37% | 396 | aaagaaagatagagcaagaca (SEQ ID NO: 56) |
| hVEGF#8 | 32% | 450 | aaagcatttgtttgtacaaga (SEQ ID NO: 83) |
| hVEGF#9 | 42% | 467 | aagatccgcagacgtgtaaat (SEQ ID NO: 84) |
| hVEGF#10 | 53% | 498 | aaacacacactcgcgttgcaa (SEQ ID NO: 85) |
| Cand5 | 63% | 328 | aaacctcaccaaggccagcac (SEQ ID NO: 51) |

*siRNA Transfection and Hypoxia Induction In Vitro.* Human 293 cells were cultured in 24 well plates at 37°C with 5% CO₂ overnight. The next day, transfections were performed when cells were about 50%-70% confluent. Cells were transfected with siRNAs directed against human VEGF. siRNAs were mixed in a CaPi reagent and added to 20 µl of 250 mM CaCl₂ solution. The siRNA/CaCl₂ mixture was added drop-wise to 20 µl of 2X Hanks Balanced Salt Solution (HBS), while mixing by vortex. The siRNA/CaCl₂/HBS complex was added directly to the medium in each well (300 µL/well). After a 4-hour incubation at 37°C, the medium was removed, and the cells were further incubated with 10% DMSO-containing serum-free medium (300 µL/well at room temperature for 1-2 minutes). This medium was then removed, and the cells were fed again with growth medium (500 µL/well). Negative controls included transfection reagent lacking siRNA and nonspecific siRNA (EGFP1 siRNA). For screening experiments siRNAs were used at a concentration of 25nM. For dose response experiments, siRNAs were used at concentrations of 1 nM, 5nM and 25nM. Hypoxia was induced with desferrioxamine at a final concentration of 130 uM 4 hours after transfection was performed. Desferrioxamine mimics a hypoxic state, as it is proposed to disrupt normal oxygen-sensing pathways in mammalian cells by inhibiting heme-Fe2+ interactions.

*VEGF Protein Quantification.* Approximately 48 hours post transfection, the supernatant was removed from all wells and a human VEGF ELISA (R & D systems, Minneapolis, MN) was performed on the 293 cells as described in the Quantikine human VEGF ELISA protocol. VEGF-specific Antibody was added to each well causing color development in proportion to the amount of VEGF bound to the plate. ELISA results were read on an AD340 plate reader at 450 nm (Beckman Coulter).

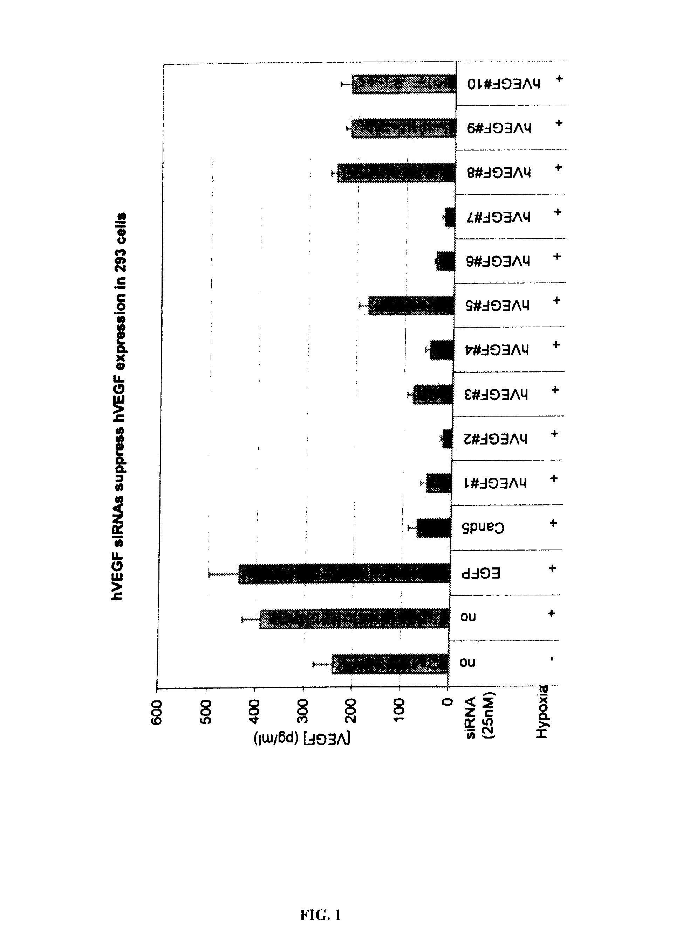

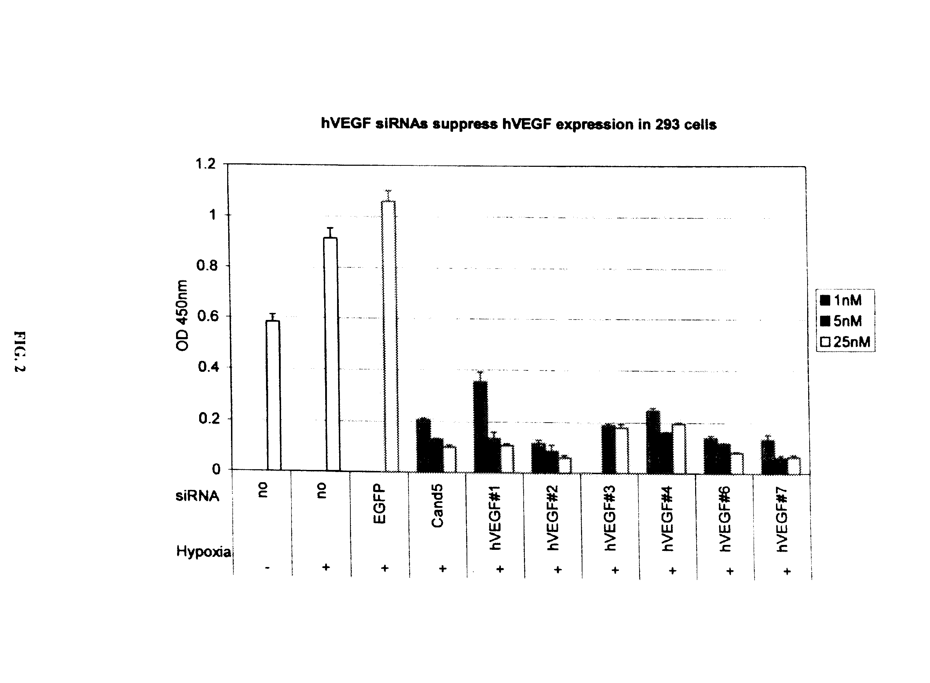

*Results. Human VEGF siRNAs Suppresses Hypoxia-Induced Up-regulation of Human VEGF Protein in 293 Cells.* Human VEGF was upregulated by the desferrioxamine-mediated induction of hypoxia. Readings of OD 450nm reflected the human VEGF protein levels in cell samples. The hypoxia-induced increase of hVEGF protein levels were significantly reduced in cells transfected with all of the human VEGF siRNAs (Figure 1). No effect on VEGF levels were observed with transfections with nonspecific siRNA (EGFP siRNA) or mock transfections without siRNA. Dose response studies were performed on Cand5, hVEGF#1, hVEGF#2, hVEGF#3, hVEGF#4, hVEGF#6 and hVEGF#7 (Figure 2).

**Example 3 - *In Vivo* RNA Interference of VEGF in Humans with ARMD using Anti-VEGF *siRNA***

Study Design. A total of 110 study subjects participated fully at 28 clinical sites (of the 129 initially enrolled). Thirty-four (34) subjects were randomized to treatment with Cand5 at 0.2 mg, while 39 subjects were assigned to treatment with Cand5 at 1.5 mg and 37 subjects received treatment with Cand5 3.0 mg.

Subjects were screened for subfoveal choroidal neovascularization (CNV) associated with ARMD using screening procedures which included manifest refraction, best corrected visual acuity, slit lamp examination, indirect ophthalmoscopic examination, fluorescein angiography, fundus photography, and ocular coherence tomography ("OCT"). Subjects were eligible for the study if all of the following criteria were met:
1. Subfoveal classic, predominantly classic, or minimally classic lesions, secondary to ARMD, with a total lesion size (including blood, atrophy/scar and neovascularization) of ≤ 12 total disc areas (30.48 mm²), of which at least 50% are active CNV and subfoveal. Minimally classic lesions were to have evidence of lesion activity defined as:
   a. The presence of subretinal hemorrhage and/or fluid and/or lipid OR
   b. Loss of one or more lines of vision (ETDRS or equivalent; *Early Treatment Diabetic Retinopathy Study* visual acuity charts) during the past 12 weeks OR
   c. FA documented lesion growth by ≥ 10% during the past 12 weeks;
2. ETDRS best corrected visual acuity of 64 to 24 letters (20/50 to 20/320 Snellen Equivalent) in the study eye;
3. Subretinal hemorrhage (if any) no more than 50% of total lesion size, and may not involve the subfoveal space;
5. ≥50 years of age;
6. Provide written informed consent form;
7. Willing and able to return for scheduled follow-up examinations.
8. For females, post-menopausal ≥1 year or surgically sterilized.

All subjects received two intravitreal injections of 100µL of Cand5 of the assigned dose. The injections were administered six weeks apart, on Day 0 and Week 6. Subjects were scheduled to return for examination 3 and 6 weeks after the first injection, and 3, 6, 9, and 12 weeks after the second injection of the double-masked investigational product. Phone contact was to be made with all subjects at 1 day and 1 week after each injection. Additional follow up visits were performed at 30, 52, and 104 weeks after the first injection.

For efficacy endpoints, the Last Observed Carried Forward (LOCF) was to be used as an imputation method for subjects with missing values. For subjects who received rescue therapy, the last observed efficacy values prior to the rescue therapy were to be used as the LOCF value. In addition to the LOCF analyses, the analyses on the available observed data without imputation was performed. Unless otherwise specified, analyses is on the available observed data.

Study Results. A summary of key efficacy and safety outcomes is provided in Table 3. As shown, distance visual acuity was stable from baseline to 12 weeks in approximately 78% of the study cohort; this increased to approximately 83% of the study cohort when considering the period from 3 to 15 weeks. CNV size changed only minimally, suggesting stabilization of the lesion. This was further reflected in the decrease in percent of lesion represented by CNV (also see **Table 11**).

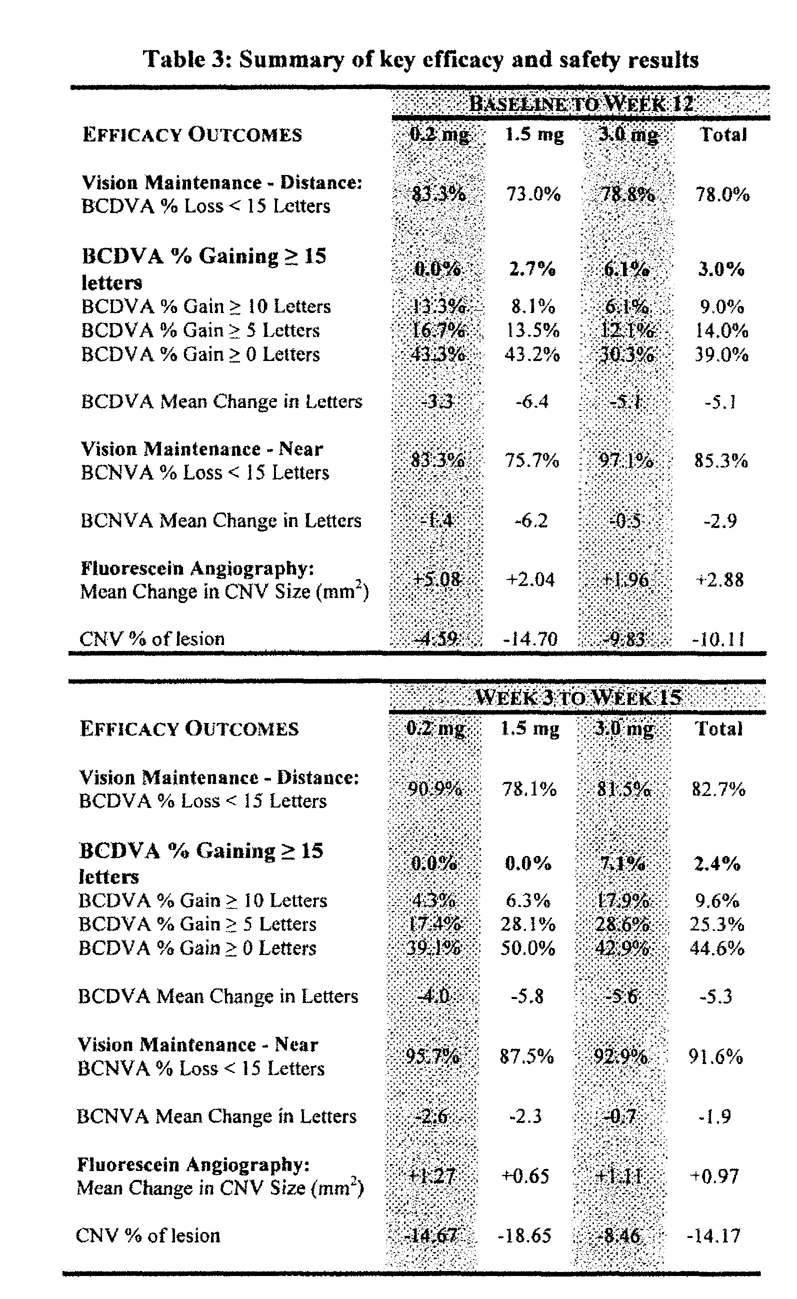

In reviewing the results, it is important to note that Cand5 induces the degradation of VEGF mRNA and thus prevents the production of new VEGF protein, but is not a VEGF antagonist and therefore may have no action on previously produced VEGF protein. The efficacy of Cand5 in inhibiting VEGF production may not be immediately manifest as a result of previously expressed VEGF present in the retinal tissue.

Given these considerations, analysis is presented with evaluation of change from baseline to 12, 15 and 18 weeks for each efficacy parameter, in addition to the analysis of mean change in letters of BCVA (best corrected visual acuity) for both distance and near visual acuity. Additionally, based on the mechanism of Cand5 action in suppression of the VEGF gene but no activity as a VEGF antagonist, evaluation of change from 3 weeks to 12, 15 and 18 weeks is presented for each key efficacy parameter. This analysis assumes turnover and degradation of VEGF present in the retinal tissues in ∼3 weeks, and allows assessment of Cand5 efficacy with regard to suppression of VEGF expression.

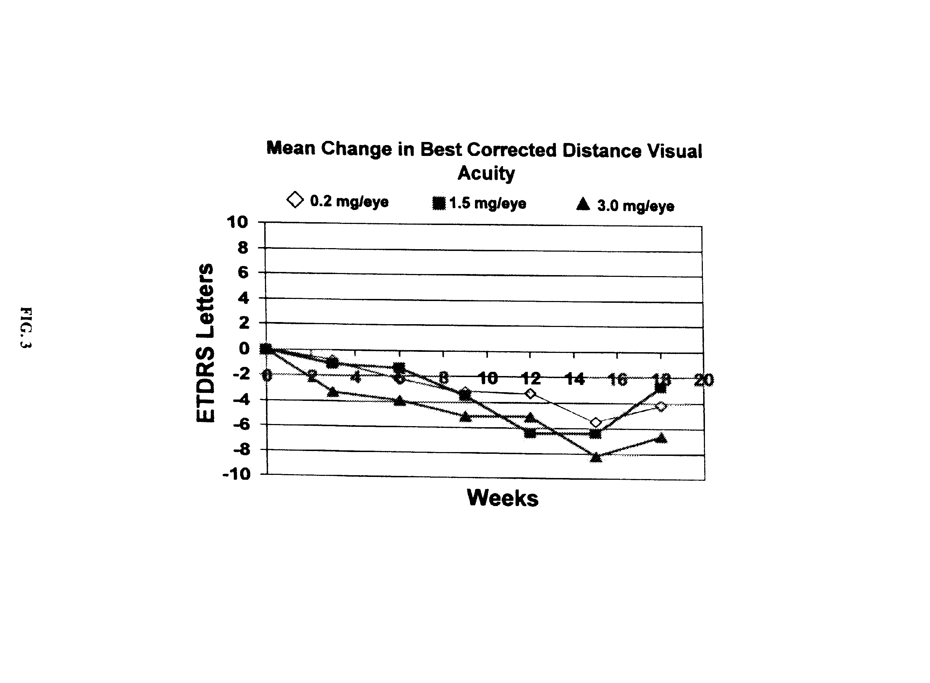

Best Corrected Distance Visual Acuity (BCDVA). The change in mean best corrected distance acuity (BCDVA) from baseline is shown in **Fig. 3****.** As shown, the mean number of letters lost increased from baseline over the course of the study, however the mean loss was less than 10 letters (<2 lines) of BCDVA for all treatment groups.

The gain in letters of best corrected distance acuity from baseline to follow-up visits at 9, 12, 15 and 18 weeks is shown in **Table 4.**

**Table 4: Summary of BCDVA increase from baseline-Subjects gaining 15, 10, 5 or any letters.**

| **BCDVA Improvement** | **0.2 mg** | **1.5 mg** | **3.0 mg** | **Fisher's Exact** | **Total** |
|---|---|---|---|---|---|
| | **n (%)** | **n (%)** | **n (%)** | **P-Value.** | **n- (%).** |
| **Week 9** | | | | | |
| N | 32 | 39 | 36 | | 107 |
| ≥ 15 letters | 1 (3.1%) | 0 (0.0%) | 1 (2.8%) | 0.5324 | 2 (1.9%) |
| ≥ 10 letters | 3 (9.4%) | 2 (5.1%) | 1 (2.8%) | 0.5095 | 6 (5.6%) |
| ≥ 5 letters | 7 (21.9%) | 5 (12,8%) | 5 (13.9%) | 0.6011 | 17 (15.9%) |

| **Week 12** | | | | | |
|---|---|---|---|---|---|
| N | 30 | 37 | 33 | | 100 |
| ≥ 0 letter | 14 (43.8%) | 16 (41.0%). | 9 (25.0%) | 0.2176 | 39 (36.4%) |
| ≥ 15 letters | 0 (0.0%) | 1 (2.7%) | 2 (6.1%) | 0.5140 | 3 (3.0%) |
| ≥ 10 letters | 4 (13.3%) | 3 (8.1%) | 2 (6.1%) | 0.6800 | 9 (9.0%) |
| ≥ 5 letters | 5 (16 7%) | 5 (13.5%) | 4 (12.1%) | 0 8768 | 14 (14.0%) |
| ≥ 0 letter | 13 (43.3%) | 16 (43.2%) | 10 (30.3%) | 0.4691 | 39 (39.0%) |

| **Week 15** | | | | | |
|---|---|---|---|---|---|
| N | 22 | 32 | 27 | | 81 |
| ≥ 15 letters | 0 (0.0%) | 1 (3.1%) | 1 (3.7%) | 1.0000 | 2 (2.5%) |
| ≥ 10 letters | 1 (4,5%) | 2 (6.3%) | 1 (3.7%) | 1.0000 | 4 (4.9%) |
| ≥ 5 letters | 4 (18.2%) | 6 (18,8%) | 3 (11.1%) | 0.7426 | 13 (16.0%) |
| ≥ 0 letter | 9 (40.9%) | 12 (375%) | 5 (18.5%) | 0,1667 | 26 (32.1%) |

| **Week 18** | | | | | |
|---|---|---|---|---|---|
| N | 19 | 25 | 20 | | 64 |
| ≥ 15 letters | 0 (0.0%) | 0 (0.0%) | 1 (5.0%) | 0.6094 | 1 (1.6%) |
| ≥ 10 letters | 2 (10.5%) | 1 (4.0%) | 1 (5.0%) | 0.6785 | 4 (6.3%) |
| ≥ 5 letters | 7(36.8%) | 5 (20.0%) | 2(10.0%) | 0.1363 | 14 (21.9%) |
| ≥ 0 letter | 7 (36.8%) | 11 (44.%) | 5 (25.0%) | 0.4127 | 23 (35.9%) |

| | | | | | |
|---|---|---|---|---|---|
| N = number of subjects with available data. n = number of subjects with the corresponding VA response in each dose group. % = n + N × 100%. | | | | | |

The same analysis of gain in letters of BCDVA from 3 weeks to the same follow-up visits is shown in **Table 5**. In the study population of subjects with relatively aggressive disease, i.e., predominantly and minimally classic but no occult, a number of subjects in each of the Cand5 dosage groups gained letters of BCDVA, with some subjects gaining as much as 10 and 15 or more letters of BCDVA, i.e., 2 to 3 or more lines.

**Table 5: Summary of BCDVA increase from week three-Subjects gaining 15, 10, 5 or any letters.**

| **BCDVA Improvement** | **0.2 mg** | **1.5 mg** | **3.0 mg** | **Fisher's Exact** | **Total** |
|---|---|---|---|---|---|
| | **n (%)** | **n (%)** | **n (%)** | **P-value** | **n (%)** |
| **Week 9** | | | | | |
| N | 32 | 39 | 36 | | 107 |
| ≥ 15 letters | 1 (3.1%) | 0 (0.0%) | 0 (0.0%) | 0.2991 | 1 (0.9%) |
| ≥ 10 letters | 1 (3.1%) | 0 (0.0%) | 2(5.6%) | 0.3960 | 3 (2.8%) |
| ≥ 5 letters | 7 (21.9%) | 5 (12.8%) | 9 (23.0%) | 0.3820 | 21 (19.6%) |
| ≥ 0 letter | 16 (50.0%) | 17 (43.6%) | 17 (47.2%) | 0,8585 | 50 (46.7%) |

| **Week 12** | | | | | |
|---|---|---|---|---|---|
| N | 30 | 37 | 33 | | 100 |
| ≥ 15 letters | 2 (6.7%) | 1 (2.7%) | 1 (3.0%) | 0.6824 | 4 (4.0%) |
| ≥ 10 letters | 2 (6.7%) | 2 (5.4%) | 4 (12.1%) | 0.6506 | 8 (8.0%) |
| ≥ 5 letters | 7 (23.3%) | 6 (16.2%) | 7 (21.2%) | 0.7787 | 20 (20.0%) |
| ≥ 0 letter | 10 (33.3%) | 13 (35.1%) | 13 (39.4%) | 0.8995 | 36 (36.0%) |

| **Week 15** | | | | | |
|---|---|---|---|---|---|
| N | 22 | 32 | 27 | | 81 |
| ≥ 15 letters | 0 (0.0%) | 1 (3.1%) | 1 (3.7%) | 1.0000 | 2 (2.5%) |
| ≥ 10 letters | 0 (0.0%) | 1 (3.1%) | 2 (7.4%) | 0.6203 | 3 (3.7%) |
| ≥ 5 letters | 4 (18.2%) | 5 (15.6%) | 6 (22.2%) | 0.9343 | 15 (18.5%) |
| ≥ 0 letter | 7 (31.8%) | 11 (34.4%) | 11 (40.7%) | 0.8386 | 29 (35.8%) |

| **Week 18** | | | | | |
|---|---|---|---|---|---|
| N | 19 | 25 | 20 | | 64 |
| ≥ 15 letters | 0 (0.0%) | 0 (0.0%) | 0 (0.0%) | | 0 (0.0%) |
| ≥ 10 letters | 1 (5.3%) | 1 (4.0%) | 2 (10.0%) | 0.8206 | 4 (6.3%) |
| ≥ 5 letters | 3 (15.8%) | 5 (20.0%) | 3 (15.0%) | 0.9201 | 11 (17.2%) |
| ≥ 0 letter | 9 (47.4%) | 9 (36.0%) | 7 (35.0%) | 0.6903 | 25 (39.1%) |

| | | | | | |
|---|---|---|---|---|---|
| N = number of subjects with available data. n = number of subjects with the corresponding VA response: in each dose group. % = n + N × 100%. | | | | | |

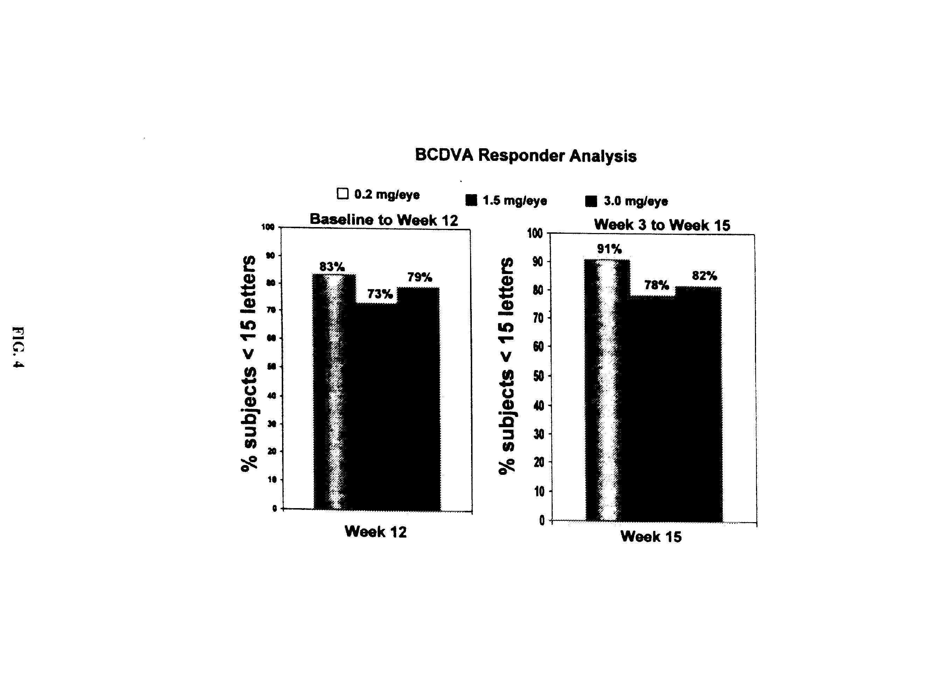

Stabilization of best corrected acuity, defined as no loss of 15 letters and considered the primary analysis in clinical trials of therapeutic agents for CNV, is shown in **Tables 6** and **7**, and **Fig. 4**. As shown in **Table 6**, approximately 85% of eyes remained stable through 9 weeks; this decreased somewhat to 78% at 12 weeks, and then remained relatively stable at 77% and 83% at 15 and 18 weeks, respectively.

**Table 6: Summary of BCDVA decrease from baseline - Subjects not losing 15, 10, 5 or any letters.**

| **BCDVA Reduction** | **0.2 mg** | **1.5 mg** | **3.0 mg** | **Fisher's Exact** | **Total** |
|---|---|---|---|---|---|
| | **n (%)** | **n (%)** | **n (%)** | **P-value** | **n (%)** |
| **Week 9** | | | | | |
| N | 32 | 39 | 36 | | 107 |
| Not losing 15 letters | 27 (84.4%) | 34(87.2%) | 30(83.3%) | 0.8902 | 91(85.0%) |
| Not losing 10 letters | 24 (75.0%) | 27 (69.2%) | 25(69.4%) | 0.8943 | 76(71.0%) |
| Not losing 5 letters | 17(53.1%) | 25(64.1%) | 22(61.1%) | 0.6415 | 64(59.8%) |
| Not losing any letters | 14(43.8%) | 16 (41.0%) | 9 (25.0%) | 0.2176 | 39(36.4%) |

| **Week 12** | | | | | |
|---|---|---|---|---|---|
| N | 30 | 37 | 33 | | 100 |
| Not losing 15 letters | 25 (83.3%) | 27 (73.0%) | 26 (78.8%) | 0.5947 | 78(78.0%) |
| Not losing 10 letters | 23(76.7%) | 22(59.5%) | 23(69.7%) | 0.3342 | 68(68.0%) |
| Not losing 5 letters | 16 (53.3%) | 19(51.4%) | 17(51.5%) | 1.0000 | 52(52.0%) |
| Not losing any letters | 13(43.3%) | 16(43.2%) | 10(30.3%) | 0.4691 | 39(39.0%) |

| **Week 15** | | | | | |
|---|---|---|---|---|---|
| N | 22 | 32 | 27 | | 81 |
| Not losing 15 letter | 18(81.8%) | 23(71.9%) | 21(77.8%) | 0.7578 | 62(76.5%) |
| Not losing 10 letters | 14(63.6%) | 19 (59.4%) | 17(63.0%) | 0.9185 | 50(61.7%) |
| Not losing 5 letters | 10(45.5%) | 17(53.1%) | 9 (33.3%) | 0.3295 | 36(44.4%) |
| Not losing any letters | 9 (40.9%) | 12 (37.5%) | 5 (18.5%) | 0.1667 | 26(32.1%) |

| **Week 18** | | | | | |
|---|---|---|---|---|---|
| N | 19 | 25 | 20 | | 64 |
| Not losing 15 letters | 16(84.2%) | 22(88.0%) | 15(75.0%) | 0.5961 | 53(82.8%) |
| Not losing 10 letters | 14 (73.7%) | 20 (80.0%) | 13(65.0%) | 0.5568 | 47 (73.4%) |
| Not losing 5 letters | 9(47.4%) | 17(68.0%) | 7(35.0%) | 0.0833 | 33 (51.6%) |
| Not losing anv letters | 7(36.8%) | 11 (44.0%) | 5(25.0%) | 0.4127 | 23(35.9%) |

| | | | | | |
|---|---|---|---|---|---|
| N = number of subjects with available data. n = number of subjects with the corresponding VA response in each dose group. % = n + N × 100%. | | | | | |

Substantially larger numbers of study subjects experienced stabilization of best corrected acuity when the change from 3 weeks is considered (**Table 7**), i.e., approximately 92% of subjects at 9 weeks, 84% at 12 weeks, and over 80% at 15 and 18 weeks. This finding is consistent with the mechanism of action of Cand5 in silencing VEGF expression, such that efficacy of Cand5 is more clearly manifest when previously expressed extracellular VEGF has been broken down (**Fig. 4**).

**Table 7: Summary of BCDVA decrease from week three-Subjects not losing 15,10,5 or any letters.**

| **BCDVA Reduction** | **0.2 mg** | **1.5 mg** | **3.0 mg** | **Ficher's Exact** | **Total** |
|---|---|---|---|---|---|
| | **n (%)** | **n (%)** | **n (%)** | **P-value** | **n (%)** |
| **Week 9** | | | | | |
| N | 32 | 39 | 36 | | 107 |
| Not losing 15 letters | 28 (87.5%) | 37 (94.9%) | 33(91.7%) | 0.5642 | 98 (91.6%) |
| Not losing 10 letters | 27(84.4%) | 34(87.2%) | 28(77.8%) | 0.5524 | 89 (83.2%) |
| Not losing 5 letters | 23 (71.9%) | 27(69.2%) | 25(69.4%) | 1.0000 | 75 (70.1%) |
| Not losing any letters | 16(50.0%) | 17(43.6%) | 17 (47.2%) | 0.8585 | 50(46.7%) |

| **Week 12** | | | | | |
|---|---|---|---|---|---|
| N | 30 | 37 | 33 | | 100 |
| Not losing 15 letters | 27(90.0%) | 29(78.4%) | 28 (84.8%) | 0.4595 | 84 (84.0%) |
| Not losing 10 letters | 24(80.0%) | 24 (64.9%) | 23(75.8%) | 0.4353 | 73(73.0%) |
| Not losing 5 letters | 17 (56.7%) | 18(48,6%) | 20 (60.6%) | 0.6092 | 55 (55.0%) |
| Not losing any letters | 10(33.3%) | 13 (35.1%) | 13 (39.4%) | 0.8995 | 36 (36.0%) |

| **Week 15** | | | | | |
|---|---|---|---|---|---|
| N | 22 | 32 | 27 | | 81 |
| Not losing 15 letters | 20(90.9%) | 25 (78.1%) | 22 (81.5%) | 0.4955 | 67(82.7%) |
| Not losing 10 letters | 17(77.3%) | 22(68.8%) | 17(63.0%) | 0.5663 | 56(69.1%) |
| Not losing 5 letters | 13 (59.1%) | 19(59.4%) | 13(48.1%) | 0.6907 | 45 (55.6%) |
| Not losing any letters | 7 (31.8%) | 11 (34.4%) | 11(40.7%) | 0.8386 | 29(35.8%) |

| **Week 18** | | | | | |
|---|---|---|---|---|---|
| N | 19 | 25 | 20 | | 64 |
| Not losing 15 letters | 16 (84.2%) | 24 (96.0%) | 16 (80.0%) | 0.2394 | 56 (87.5%) |
| Not losing 10 letters | 14 (73.7%) | 21 (94.0%) | 14 (70.0%) | 0.5334 | 49 (76.6%) |
| Not losing 5 letters | 9 (47.4%) | 16 (64.0%) | 12 (60.0%) | 0.5916 | 37 (57.8%) |
| Not losing any letters | 9 (47.4%) | 9 (36.0%) | 7 (35.0%) | 0.6903 | 25 (39.1%) |

| | | | | | |
|---|---|---|---|---|---|
| N = number of subjects with available data n = number of subjects with the corresponding VA response in each dose group. % = n + N × 100%. | | | | | |

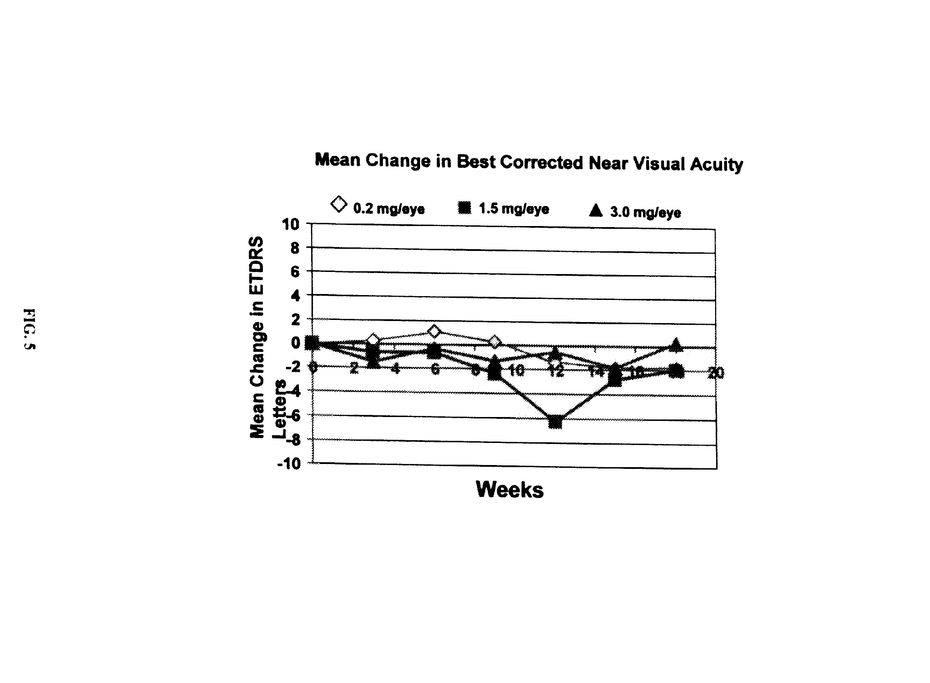

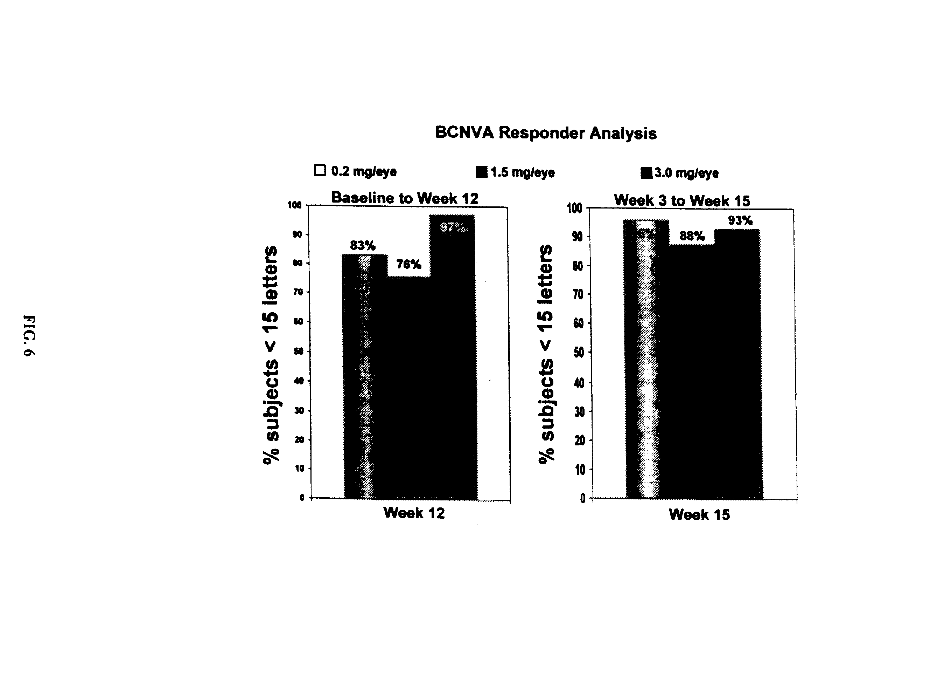

Best Corrected Near Visual Acuity. The change in letters of near visual acuity (BCNVA) from baseline over time is shown in **Tables 8** and **9**, and **Fig. 5** and **6**. Only small changes in BCNVA, generally less than 5 letters or 1 line of near vision, were observed over the course of study follow-up, suggesting good stability of the best corrected near acuity (**Fig. 5**).

Gain in letters of best corrected near acuity over time is summarized in **Table 8**. Improvement in best corrected distance acuity of 15 or more letters, i.e., 3 or more lines, was observed in approximately 5% of the study population, and remained relatively stable from week 9 through week 18. In the analysis of increase in BCDVA from baseline, a statistically significant difference between treatment groups was observed, with a greater number of subjects in the 3.0mg Cand5 group gaining letters of near acuity than in the two lower dose groups (**Table 8**; p<0.05).

**Table 8: Summary of BCNVA increase from baseline Subjects gaining 15,10,5 or any letters.**

| **BCNVA Improvement** | **0.2 mg** | **1.5 mg** | **3.0 mg** | **Fisher's Exact** | **Total** |
|---|---|---|---|---|---|
| | **n (%)** | **n (%)** | **n (%)** | **P-value** | **n (%)** |
| **Week 9** | | | | | |
| N | 32 | 39 | 36 | | 107 |
| ≥ 15 letters | 2(6.3%) | 2 (5.1%) | 4 (11.1%) | 0.6537 | 8(7.5%) |
| ≥ 10 letters | 3(9.4%) | 3 (7.7%) | 5 (13.9%) | 0.7260 | 11 (10.3%) |
| ≥ 5 letters | 10 (31.3%) | 8(20.5%) | 6(16,7%) | 0.3652 | 24(22.4%) |
| ≥ 0 letter | 21 (65.6%) | 14 (35.9%) | 12 (33.3%) | 0.0142 | 47(43.9%) |

| **Week 12** | | | | | |
|---|---|---|---|---|---|
| N | 30 | 37 | 35 | | 102 |
| ≥ 15 letters | 1 (3.3%) | 0 (0.0%) | 5 (14.3%) | 0.0182 | 6 (5.9%) |
| ≥ 10 letters | 5 (16,7%) | 5 (13.5%) | 5 (14.3%) | 0.9407 | 15 (14.7%) |
| ≥ 5 letters | 10 (33.3%) | 6 (16.2%) | 7 (20.0%) | 0.2521 | 23 (22.5%) |
| ≥ 0 letter | 15 (50,0%) | 12 (32.4%) | 13 (37.1%) | 0.3524 | 40 (39.2%) |

| **Week 15** | | | | | |
|---|---|---|---|---|---|
| N | 23 | 32 | 28 | | 83 |
| ≥ 15 letters | 0 (0.0%) | 2 (6.3%) | 4 (14.3%) | 0.1954 | 6 (7.2%) |
| ≥ 10 letters | 3 (13.0%) | 5 (15.6%) | 4 (14.3%) | 1.0000 | 12 (14.5%) |
| ≥ 5 letters | 9 (39.1%) | 9 (28.1%) | 7 (25.0%) | 0.5704 | 25 (30.1%) |
| ≥ 0 letter | 10 (43.5%) | 13 (40.6%) | 9 (32.1)%) | 0.7154 | 32 (38.6%) |

| **Week 18** | | | | | |
|---|---|---|---|---|---|
| N | 18 | 25 | 20 | | 63 |
| ≥ 15 letters | 2 (11.1%) | 1 (4.0%) | 4 (20.0%) | 0.2753 | 7 (11.1%) |
| ≥ 10 letters | 3 (16.7%) | 3 (20.0%) | 5 (25.0%) | 0.8579 | 13 (20.6%) |
| ≥ 5 letters | 4 (22.2%) | 8 (32.0%) | 5 (25.0%) | 0.8226 | 17(27.0%) |
| ≥ 0 letter | 8 (44.4%) | 13 (52.0%) | 7 (35.0%) | 0.5384 | 28 (44.4%) |

| | | | | | |
|---|---|---|---|---|---|
| N = number of subjects with available data. n = number of subjects with the corresponding VA response in each dose group. % = n + N × 100%. | | | | | |

Stabilization of best corrected near acuity, i.e., the proportion of subjects not losing 15 letters, and loss of letters of BCNVA are summarized in **Table 9** and **Fig. 6**. At week 9, over 90% of the study population had not lost 15 letters of near acuity, and only a small decrease in this proportion of responders was observed at 12, 15 and 18 weeks. Stability of the best corrected near acuity was even more notable when considering the change from 3 weeks to later visits (**Fig. 6**).

**Table 9: Summary of BCNVA decrease from baseline Subjects not losing 15, 10, 5 or any letters.**

| **BCNVA Reduction** | **0.2 mg** | **1.5 mg** | **3.0 mg** | **Fisher's Exact** | **Total** |
|---|---|---|---|---|---|
| | **n (%)** | **n (%)** | **n (%)** | **P-value** | **n (%)** |
| **Week 9** | | | | | |
| N | 32 | 39 | 36 | | 107 |
| Not losing 15 letters | 29 (90.6%) | 36 (92.3%) | 34 (94.4%) | 0.9007 | 99 (92.5%) |
| Not losing 10 letters | 25(78.1%) | 33 (84.6%) | 29 (80.6%) | 0.7814 | 87(81.3%) |
| Not losing 5 letters | 24 (75.0%) | 23 (59.0%) | 20 (55.6%) | 0.2138 | 67 (62.6%) |
| Not losing any letters | 21 (65.6%) | 14 (35.9%) | 12 (33.3%) | 0,0142 | 47 (43.9%) |

| **Week 12** | | | | | |
|---|---|---|---|---|---|
| N | 30 | 37 | 35 | | 102 |
| Not losing 15 letters | 25(83.3%) | 28 (75.7%) | 34(97.1%) | 0.0284 | 87 (85.3%) |
| Not losing 10 letters | 23 (76.7%) | 23(62.2%) | 30(85.7%) | 0.0758 | 76 (74.5%) |
| Not losing 5 letters | 19 (63.3%) | 16 (43.2%) | 18 (51.4%) | 0.2769 | 53 (52.0%) |
| Not losing any letters | 15(50,0%) | 12(32.4%) | 13 (37.1%) | 0.3524 | 40 (39.2%) |

| **Week 15** | | | | | |
|---|---|---|---|---|---|
| N | 23 | 32 | 28 | | 83 |
| Not losing 15 letters | 20 (87.0%) | 28 (87.5%) | 25 (89.3%) | 1.0000 | 73 (88.0%) |
| Not losing 10 letters | 18 (78.3%) | 21 (65.6%) | 19 (67,9%) | 0.6245 | 58 (69.9%) |
| Not losing 5 letters | 17 (73.9%) | 18 (56.3%) | 14 (30.0%) | 0.2035 | 49 (59.0%) |
| Not losing any letters | 10(43.5%) | 13 (40.6%) | 9 (32.1%) | 0.7154 | 32 (38.6%) |

| **Week 18** | | | | | |
|---|---|---|---|---|---|
| N | 18 | 25 | 20 | | 63 |
| Not losing 15 letters | 15 (83.3%) | 22 (88.0%) | 18 (90.0%) | 0.8015 | 55 (87.3%) |
| Not losing 10 letters | 13 (72.2%) | 17 (68.0%) | 16 (80.0%) | 0.7216 | 46 (73.0%) |
| Not losing 5 letters | 11 (61,1%) | 13 (52.0%) | 12 (60.0%) | 0.8520 | 36(57.1%) |
| Not losing any letters | 8(44.4%) | 13 (52.0%) | 7 (35.0%) | 0.5384 | 28 (44.4%) |

| | | | | | |
|---|---|---|---|---|---|
| N = number of subjects with available data. n = number of subjects with the corresponding VA response in each dose group. % = n + N × 100%. | | | | | |

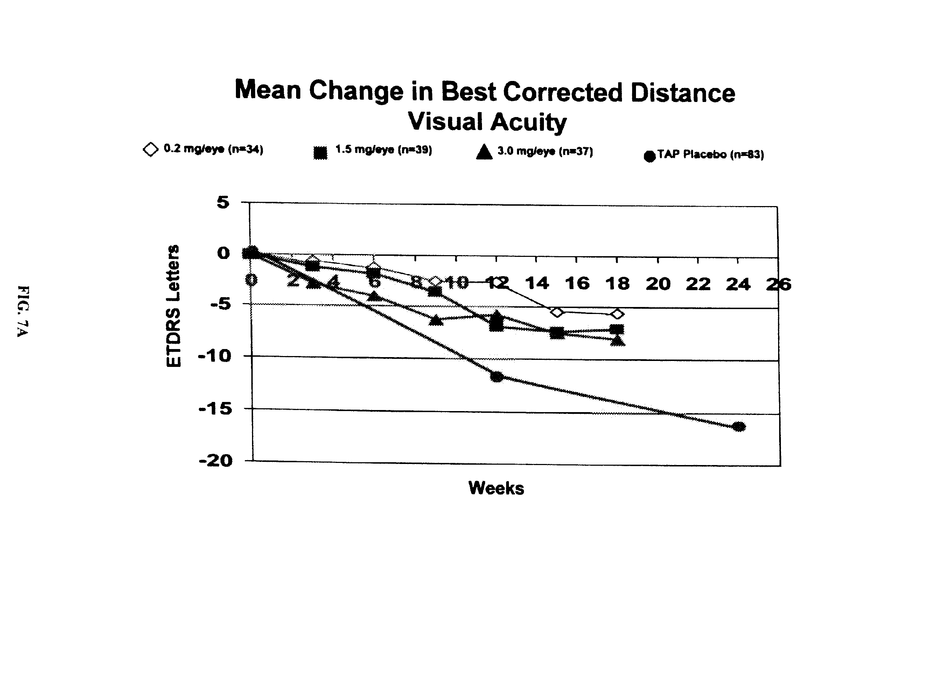

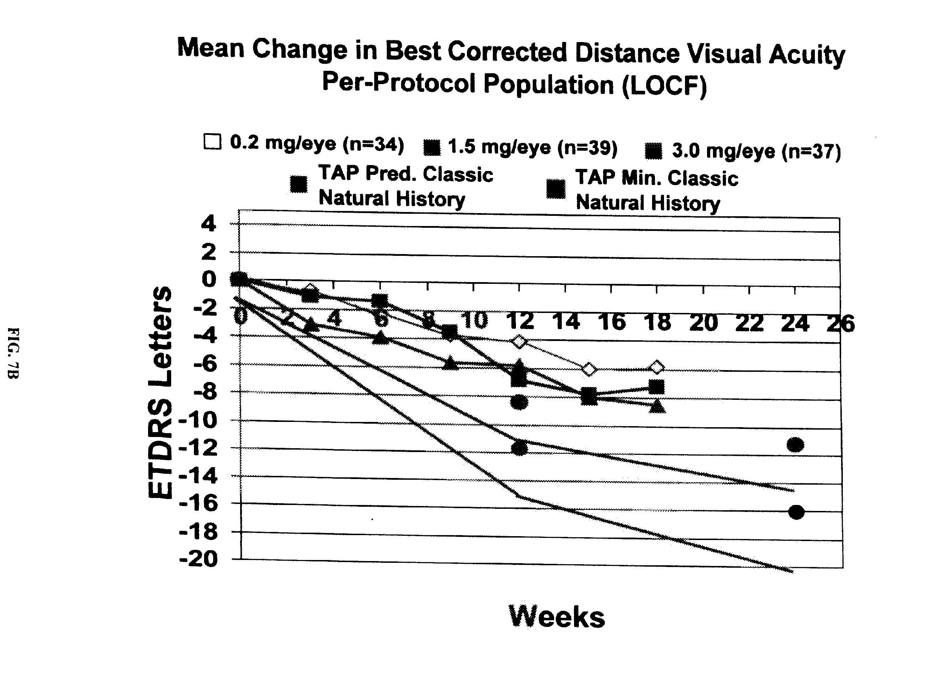

Given the apparent clinical effect of Cand5 administered at a dose of 0.2 mg, leaving absent a proper control group, comparison to published data on an untreated or sham control is of interest in assessing the clinical benefit of Cand5 versus a natural history. The Treatment of ARMD with Photodynamic Therapy (TAP: Blinder KJ et al. (2003) TAP and VIP report No. 1, Amer J Ophth 136:407-18) placebo group is particularly appropriate for comparison as its subject population is most similar to the predominantly classic population in this trial of Cand5. It could be argued that this is a conservative comparison as the subject population in the TAP trial had no other therapeutic options available for treatment of ARMD, allowing for a completely treatment naïve population not subject to investigator bias during enrollment. Investigator bias towards treating subjects with competing therapies or enrolling subjects into other clinical trials may have led to a population in this current study that was skewed towards subjects who were unlikely to have done well on other treatment. Placebo subjects in the TAP study, with predominantly classic disease, had lost 11.7 letters at 3 months (Bressler NM et al. (2002) Arch Ophthalmol. 120:1443-54), compared to a loss of 5.5 letters in the corresponding Cand5 population (**Fig. 7A** **and** **7B** (LOCF)). A total of 69.0% of TAP predominantly classic placebo subjects maintained vision (loss of <15 letters), in contrast to 78.3% of subjects in the current study.

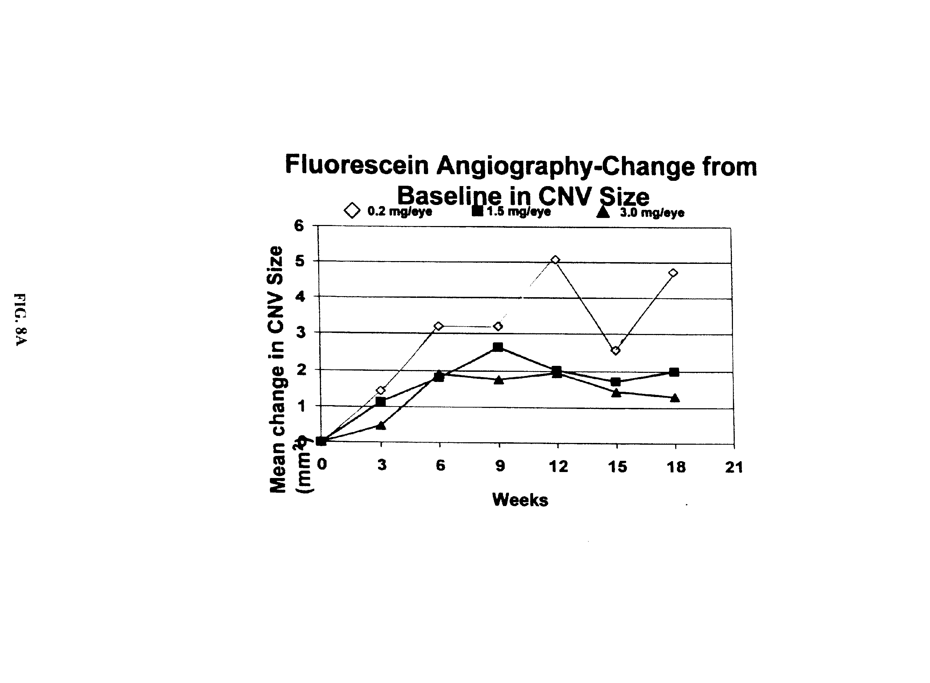

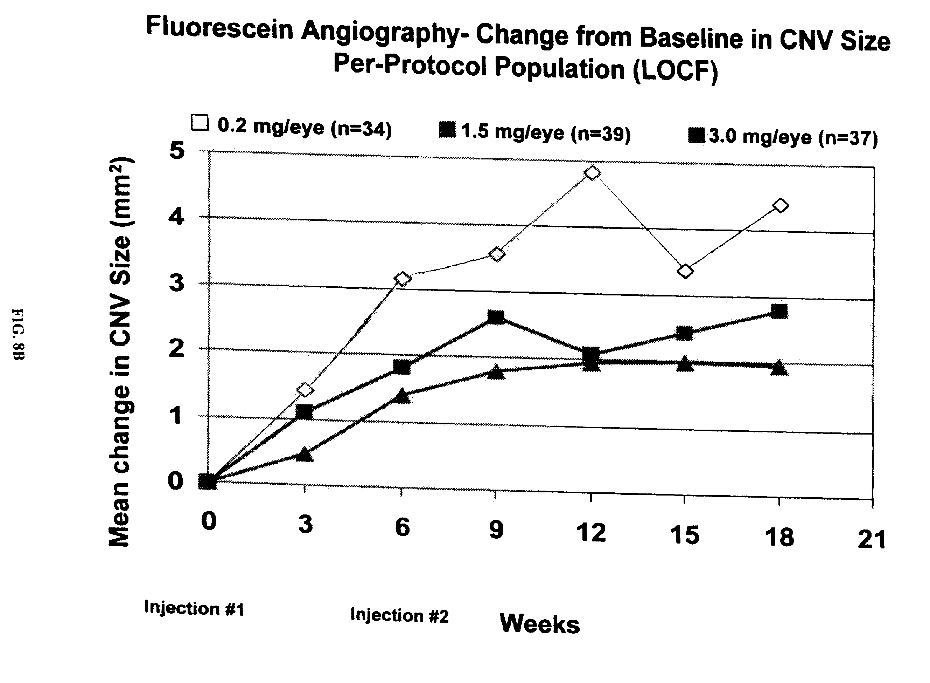

CNV Size. Change from baseline in mean size of Choroidal Neovascularization (CNV) is shown in **Fig. 8A** **and** **8B** (LOCF). VEGF has been shown to be both necessary and sufficient for the growth of CNV in animal models (Adamis AP et al. (1996) Arch Ophthalmol 114:66-71), and therefore is expected to be the most VEGF responsive component of the lesion. It is noteworthy to point out that there is evidence of a dose effect trend, that while not statistically significant is apparent at all visits in the growth of CNV from baseline.

Total Lesion Size. Change from baseline in total lesion size is shown in **Table 10.** While the more pertinent parameters are represented by change in CNV size and change in the proportion of the lesion represented by CNV, information on total lesion size has utility as background for the analysis of change in CNV as the percent of the overall lesion dimensions. It is also noteworthy that the change in total lesion size was smaller in the highest dosage group than in the 0.2 mg group and the 1.5 mg group, especially at later time points, although these differences were not statistically significant.

**Table 10: Fluoroscein Angiography - total lesion size.**

| **Visit** | **Statistics** | **0.2 mg** | **1.5 mg** | **3.0 mg** | **Total** |
|---|---|---|---|---|---|
| Week 3 | N | 34 | 38 | 37 | 109 |
| | Mean | 2.11 | 1.17 | 1.81 | 1.68 |
| | 95% CI | 0.73, 3.50 | 0.07, 2.27 | 1.10, 2.51 | 1.07, 2.29 |
| | SD | 3.97 | 3.35 | 2.12 | 3.21 |
| | Median | 1.29 | 1.I8 | 1.62 | 1.38 |
| | Range | -5.2, 17.0 | -14.1,8.4 | -5.1, 6.3 | -14.1, 17.0 |
| Week 6 Pre-injection | N | 33 | 39 | 35 | 107 |
| | Mean | 3.61 | 2.15 | 2.60 | 2.74 |
| | 95% Cl | 1.73, 5.49 | 0.80, 3.50 | 1.79, 3.40 | 1.96, 3.53 |
| | SD | 5.30 | 4.16 | 2.34 | 4.10 |
| | Median | 1.93 | 1.91 | 2.46 | 2.13 |
| | Range | -5.7, 20.8 | -13.0, 11.8 | -1.6, 7.7 | -130, 20.8 |
| Week 9 | N | 31 | 38 | 35 | 104 |
| | Mean | 4.03 | 3.13 | 2.87 | 3.31 |
| | 95% Cl | 2.24, 5.83 | 1.28, 4.99 | 1.87, 3.87 | 2.41, 4.21 |
| | SD | 4.89 | 5.65 | 2.91 | 4 63 |
| | Median | 2.94 | 2.70 | 2.68 | 2.73 |
| | Range | -3.1, 18.0 | -12.8, 9.2 | -4.6, 8.6 | -12.8, 19.2 |
| Week 12 | N | 29 | 37 | 35 | 101 |
| | Mean | 5.92 | 4.56 | 3.32 | 4.52 |
| | 95% Cl | 3.22, 8.62 | 2.22, 6.90 | 2.16, 4.48 | 3.32, 5.72 |
| | SD | 7.09 | 7.03 | 3.37 | 6.07 |
| | Median | 3.04 | 3.00 | 2.99 | 3.00 |
| | Range | -1.9, 23.9 | -13.2, 20.5 | -3.0, 11.9 | -13.2, 23.9 |
| Week 15 | N | 23 | 33 | 28 | 84 |
| | Mean | 6.64 | 4.87 | 3.42 | 4.87 |
| | 95% Cl | 3.20,10.07 | 2.40, 7.34 | 1.83, 5.01 | 3.46,6.29 |
| | SD | 7.95 | 6.98 | 4.09 | 6.52 |
| | Median | 3.72 | 3.89 | 2.88 | 3.43 |
| | Range | -2.9, 24.3 | -9.4, 30.6 | -5.6, 11.8 | -9.4, 30.6 |
| Week 18 | N | 19 | 24 | 18 | 61 |
| | Mean | 9.13 | 6.78 | 4.19 | 6.75 |
| | 95% Cl | 4.79, 13.47 | 2.06, 11.49 | 0.99, 7.38 | 4.35, 9.14 |
| | SD | 9.01 | 11.17 | 6.43 | 9.36 |
| | Median | 6.20 | 4 92 | 3.96 | 4.90 |
| | Range | -3.9, 24.4 | -6.7, 50.1 | -8.5, 22.6 | -8.5, 50,1 |

| | | | | | |
|---|---|---|---|---|---|
| Baseline = the most recent non-missing data prior to the treatment injection at Day 0. N = number of subjects with available data. | | | | | |

CNV Percent of Total Lesion Size. The change in the proportion of the total lesion represented by CNV is shown in **Table 11**. As shown, there was an overall decrease in the CNV percent of total lesion size in all three treatment groups, with increasing magnitude of this change from week 3 through week 18.

**Table 11: Fluoroscein Angiography change from baseline-choroidal neovascularization % of lesion.**

| **Visit:** | **Statistics** | **0.2 mg** | **1.5 mg** | **3.0 mg** | **Total** |
|---|---|---|---|---|---|
| Week 3 | N | 34 | 38 | 37 | 109 |
| | Mean | -4.03 | 1.53 | -7.57 | -3.29 |
| | 93% Cl | -11.91, 3.85 | -1.30, 4.35 | -14.29, -0.85 | -6.75, 0 16 |
| | SD | 22.57 | 8.60 | 20.15 | 18.19 |
| | Median | 0.00 | 0.00 | 0.00 | 0.00 |
| | Range | -100.0, 28.0 | -17.0, 28.5 | -82.5, 14.5 | -100.0, 28.5 |
| Week 6 Pre-injection | N | 33 | 39 | 35 | 107 |
| | Mean | -1.36 | -0.73 | -3.40 | -1.80 |
| | 93% Cl | -8.43, 5.71 | -7.15, 5.69 | -7.70, 0.90 | -5.19, 1.59 |
| | SD | 19.94 | 19.81 | 12.51 | 17.61 |
| | Median | 0.00 | 0.00 | 0.00 | 0.00 |
| | Range | -100.0, 27.5 | -100.0, 37.5 | -61.5, 21.0 | -100.0, 37.5 |
| Week 9 | N | 31 | 38 | 35 | 104 |
| | Mean | -3.40 | -3.41 | -5.56 | -4.13 |
| | 95% Cl | -12.00, 5.19 | -11.97, 5.15 | -13.08, 1,97 | -8.74, 0.48 |
| | SD | 23.42 | 26.04 | 21.90 | 23.72 |
| | Median | 0.00 | 0.00 | 0.00 | 0.00 |
| | Range | -100.0, 25.0 | -100.0, 28,5 | -82.5, 49.5 | -100.0, 49.5 |
| Week 12 | N | 29 | 37 | 35 | 101 |
| | Mean | -4.59 | -14.70 | -983 | -10.1 |
| | 95% Cl | -15.90, 6.73 | -26.56, -2.85 | -19.31, -0.35 | -16.28, -3.93 |
| | SD | 29.76 | 35.55 | 27.59 | 31.28 |
| | Median | 0.00 | 0.00 | 0.00 | 0.00 |
| | Range | -100.0, 36.0 | -100.0, 34.0 | -100.0, 22.5 | -100.0, 36.0 |
| Week 15 | N | 23 | 33 | 28 | 84 |
| | Mean | -23.04 | -16.59 | -15.52 | -18.00 |
| | 95% Cl | -41.99, -4.10 | -30.46, -273 | -28.25, -2.79 | -26.29, -9.71 |
| | SD | 43.80 | 39.10 | 32.83 | 38.19 |
| | Median | -2.50 | 0.00 | 0.00 | 0.00 |
| | Range | -100.0, 32.0 | -100.0, 34.0 | -86.5, 49.5 | -100.0, 49.5 |
| Week 18 | N | 19 | 24 | 18 | 61 |
| | Mean | -25.16 | -25.17 | -15.11 | -22.20 |
| | 95% Cl | -45.17, -5.15 | -42.53, -7.81 | -30.25, 0.03 | -31.94, -12.45 |
| | SD | 41.51 | 41.11 | 30.44 | 38.06 |
| | Median | -1.00 | 0.00 | -0.25 | 0.00 |
| | Range | -100.0, 12.5 | -100.0, 16.0 | -82.5, 49.5 | -100.0, 49.5 |

| | | | | | |
|---|---|---|---|---|---|
| Baseline = the most recent non-missing data prior to the treatment injection at Day 0. N = number of subjects with available data. | | | | | |

The change in CNV % of lesion from week 3 to weeks 12, 15 and 18 is shown in **Table 12**. Consistent with the analysis of change from baseline, there is a decrease over time in the % of lesion represented by CNV, indicating efficacy of Cand5 in reducing the CNV size based on percent of the overall lesion. This effect was observed for the total study cohort as well as for any lesion type.

**Table 12: Fluoroscein angiography change from week three-Choroidal neovascularization % of lesion.**

| **Visit** | **Statistics** | **0.2 mg** | **1.5 mg** | **3.0 mg** | **Total** |
|---|---|---|---|---|---|
| Week 6 Pre-injection | N | 33 | 39 | 35 | 107 |
| | Mean | 2.79 | -2.22 | 2.23 | 0.78 |
| | 95% CI | -0.56, 6.14 | -8.12, 3.68 | -1.86, 6.32 | -1.91, 3.47 |
| | SD | 9.45 | 18.20 | 11.90 | 14.02 |
| | Median | 0.00 | 0.00 | 0.00 | 0.00 |
| | Range | -6.5, 34.0 | -100.0, 22.0 | -14.0,61.5 | -100.0, 61.5 |
| Week 9 | N | 31 | 38 | 35 | 104 |
| | Mean | -0.45 | -4.93 | 0.44 | -1.79 |
| | 95% CI | -4.31, 3.41 | -13.13, 3.26 | -5.97, 6.86 | -5.56, 1.99 |
| | SD | 10.52 | 24.94 | 18.68 | 19.41 |
| | Median | 0.00 | 0.00 | 0.00 | 0.00 |
| | Range | -34.5, 23.5 | -100.0, 28.5 | -69.5, 55.5 | -100.0, 55.5 |
| Week !2 | N | 29 | 37 | 35 | 101 |
| | Mean | 1.03 | -16.24 | -1.74 | -6.26 |
| | 95% Cl | -6.21, 8.28 | -28.22, -4.26 | -11.92, 8.44 | -12.26, -0.26 |
| | SD | 19.04 | 35.93 | 29.64 | 30.40 |
| | Median | 0.00 | 0.00 | 0.00 | 0.00 |
| | Range | -67.5, 3.0 | -100.0, 34.5 | -100.0, 72.5 | -100.0, 72.5 |
| Week 15 | N | 23 | 33 | 28 | 84 |
| | Mean | -14.67 | -18.65 | -8.46 | -14.17 |
| | 95% Cl | -29.19, -0.16 | -32.02, -5.28 | -20,52, 3.59 | -21.62, -6.71 |
| | SD | 33.56 | 37.71 | 31.09 | 34.36 |
| | Median | -2.00 | 0.00 | 0.00 | 0.00 |
| | Range | -100.0, 23.5 | -100.0, 34.5 | -74.5, 55.5 | -100.0, 55.5 |
| Week 18 | N | 19 | 24 | 18 | 61 |
| | Mean | -14.45 | -26.96 | -4.61 | -16.47 |
| | 95% Cl | -28.68, -0.22 | -43.73, -10.18 | -19.76, 10.54 | -25.40, -7.53 |
| | SD | 29.52 | 39.72 | 30.47 | 34.88 |
| | Median | 0.00 | 0.00 | 0.00 | 0.00 |
| | Range | -100.0, 16.5 | -100.0, 9.0 | -67.0, 60.0 | -100.0, 60.0 |

| | | | | | |
|---|---|---|---|---|---|
| N = number of subjects with available data. | | | | | |

Summary and Discussion. Cand5 may act by silencing VEGF expression, but is not a VEGF antagonist and has no action on previously produced VEGF protein. In subjects with wet ARMD, the increased circulating levels of VEGF in various tissues of the eye continue to promote neovascularization and vascular permeability in the absence of new VEGF production until the protein is turned over by receptor binding and/or natural catabolic processes. The efficacy of Cand5 in inhibiting VEGF production may not be immediately manifest until this "turnover" of protein occurs. As this extracellular VEGF protein population turns over, new VEGF production would be inhibited by Cand5.

Thus, the analysis of change from 3 weeks to 12, 15 and 18 weeks for each key outcome may represent a more pharmacologically relevant time window for observing Cand5 efficacy. This analysis assumes turnover and degradation of extracellular VEGF present in the retinal tissues in approximately 3 weeks, although this has not been definitively established.

While no significant between-group differences were identified, several important trends in key outcomes are noteworthy. Specifically, distance visual acuity was stable from Baseline to Week 12 in approximately 78% of the study subjects; this increased to approximately 83% of the study cohort when considering the period from Week 3 to Week 15. This visual acuity stabilization of 83% was maintained through the Week 18 visit, which was 12 weeks since the last treatment with Cand5.

CNV size changed only minimally in this 18 week period, suggesting stabilization of the lesion. Importantly, a fairly clear dose effect was observed in CNV lesion size, where the growth in CNV size was greatest in the 0.2 mg dose of Cand5, somewhat lower in the 1.5 mg group, and minimal in the eyes treated with Cand5 at a dose of 3.0 mg. VEGF has been shown to be both necessary and sufficient for the growth of CNV in animal models [3], and therefore CNV is expected to be the most VEGF responsive component of the lesion. Thus, this result is a particularly appropriate marker for the Cand5 effect. While the difference between change in CNV size was not statistically significant from Baseline to Week 12 (p=0.0582), it is expected that this would be further demonstrated when investigated in a larger population.

These findings in visual acuity and CNV size stabilization are particularly noteworthy given the characteristics of the study population with regard to CNV lesion type. To allow evaluation of Cand5 efficacy following 12 weeks of treatment, subjects with aggressive disease were enrolled. The study cohort comprised 64% predominantly classic lesions and 36% minimally classic lesions; subjects with occult disease were excluded from enrollment. Minimally classic lesions were required to be active, and over the 3 months prior to enrollment, an increase in lesion size of ≥10% or a loss of ≥1 line of BCDVA were required. Finally, multiple previous treatments were allowed, i.e., up to two injections of Macugen,^{®} one triamcinolone injection, and one PDT treatment. In this study, 24% of subjects had been previously treated for ARMD in their study eye. It is interesting to note that 21% of lesions were identified as having characteristics of retinal angiomatous profileration (RAP) as determined by the Digital Angiography Reading Center. The unanticipated incidence of highly aggressive RAP lesions led to the introduction of an additional randomization stratification during the course of the study. Notwithstanding this severely affected population, Cand5 appeared to have efficacy in all dose groups.

It can be concluded that Cand5 is safe and efficacious at doses up to 3.0 mg administered by intravitreal injection.

Based upon the foregoing, it is also submitted that it may be advantageous to combine the administration of Cand5 with a second therapy. For example, an acute, initial therapy consisting of the administration of Lucentis on a monthly basis for the first 3 months or longer and administration of Cand5 as a maintenance therapy, which does not appear to impact the pre-existing VEGF.

### Example 4 - In Vivo RNA Interference of VEGF in Humans with DME using Anti-VEGF siRNA

Study Design. Forty-eight (48) subjects were randomized to treatment with Cand5 at 0.2 mg (18), at 1.5 mg (12) and 3.0 mg (18). The injections were administered four weeks apart, on Day 0, Week 4 and Week 8. Subjects were at least 21 years old and diagnosed with Type 1 or Type 2 diabetes. The subjects eye(s) had macular edema involving the center of the macula on OCT of at least 250 microns or more in the central subfield, with no evidence of tractional etiology, and CSME on ophthalmoscopic examination, a best-corrected visual acuity with letter scores between .69 and 24 and snellen equivalent (20/40-20/230). Patients were excluded if they had chronic renal failure, unstable medical status (HbA1C of more than 10%), PRP within 12 weeks of inclusion or anticipated RPR, focal laser within 12 weeks of inclusion or use of steroids within 6 months of inclusion.

The baseline age was 62.7 years, 56% female and 44% male, 70% Caucasian and 30% non-Caucasian. In the fellow eye, 76.7% had DME and 23.3% did not have DME. The mean visual acuity was 57.3 letters (20/63). Using the international clinical diabetic retinopathy disease severity scale 6 were mild non-proliferative, 18 were moderate non-proliferative, 8 were severe non-proliferative and 16 were proliferative.

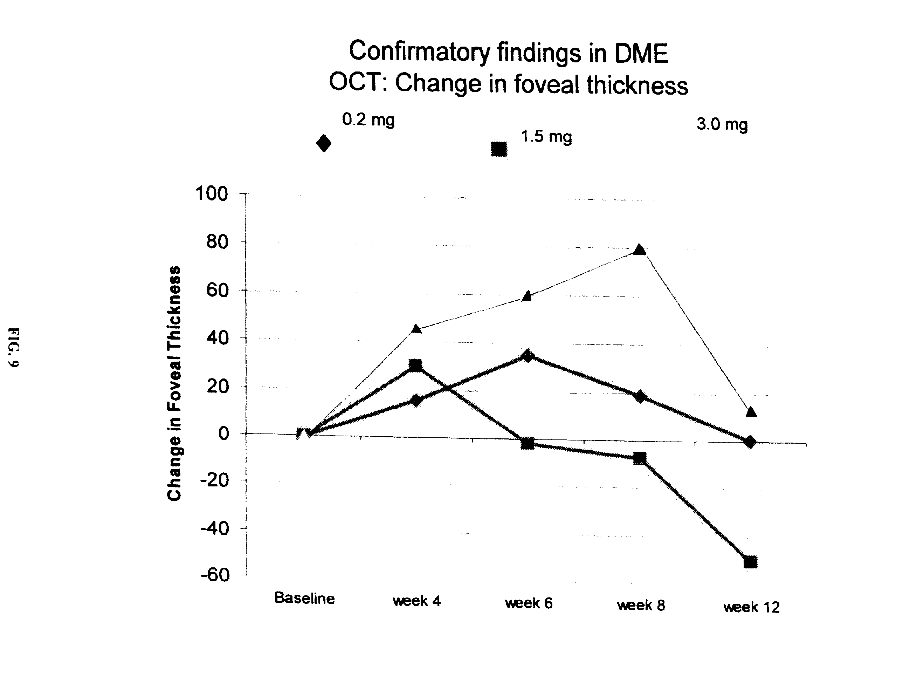

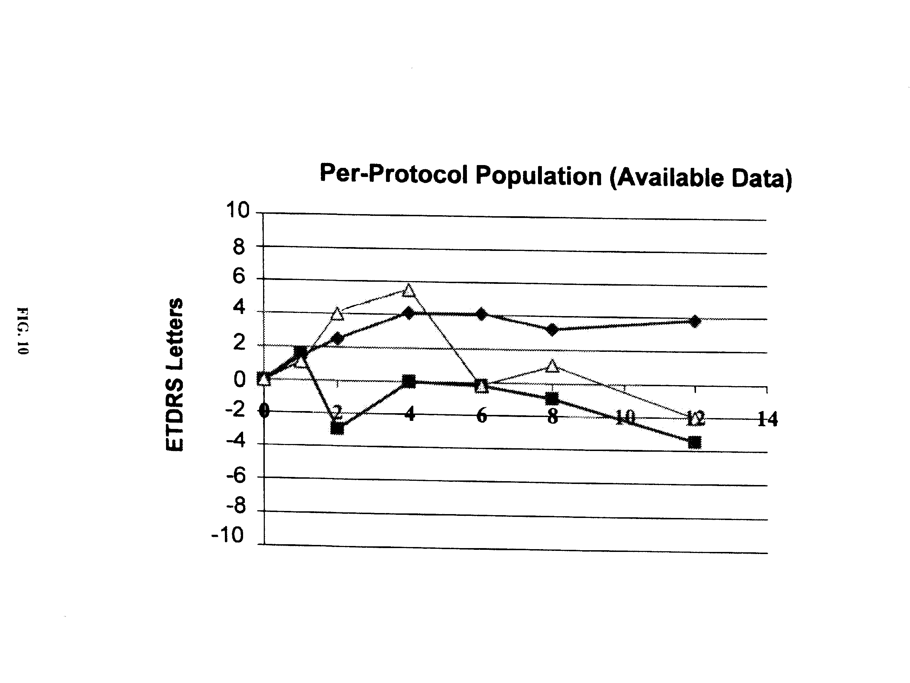

Samples were taken at 30 minutes, 3 hours and 24 hours post intravitreal injection. No plasma samples contained detectable levels of Cand5. The primary efficacy endpoint was change in OCT thickness at 12 weeks and the secondary efficacy endpoint was change in visual acuity at 12 weeks. The primary endpoint (OCT) was measured as a change in retinal thickness compared to mean thickness in the central subfield. The mean change from baseline at 12 weeks, after 3 doses was -8.9 um (range -547um to 310 um), which is further depicted in **Figure 9**. The secondary endpoint was analyzed as the mean change in corrected acuity in letters. The mean change from baseline at 12 weeks, after 3 doses was 0.1 letters (range -39 letters to 21 letters), which is further depicted in **Figure 10**.

Cand5 is safe and well tolerate in patients with DME. At three months, after 3 doses, there was minimal change in mean OCT thickness and in visual acuity, however, subsets of patients did well supporting a biologic effect in DME. As observed in AMD, pools of pre-existing extracellular VEGF do not appear to be managed, but the production of new VEGF is inhibited. The ultimate effect on the endpoints, therefore, require time to be observed.
The present invention provides a method of stabilizing visual acuity in a subject comprising administering to the subject an effective amount of an siRNA comprising a sense RNA strand of SEQ ID NO: 77 and an antisense RNA strand of SEQ ID NO: 78.
The sense and antisense RNA strands can be stabilized against nuclease degradation.
The siRNA can be administered by an intraocular administration route, optionally the intraocular administration route is selected from intravitreal, intraretinal, subretinal, subtenon, peri- and retro-orbital, trans-corneal and trans-scleral administration.
The above method of the invention can further comprise administering a VEGF antagonist. Optionally the VEGF antagonist is a monoclonal antibody targeting human VEGF selected from bevacizumab and ranibizumab, preferably the VEGF antagonist is ranibizumab.
In the above method of the invention, the effective amount can be from about 0.5 mg to about 5 mg. The effective amount can be about 2.5 mg.
The effective amount of said VEGF siRNA can be administered every four weeks. The effective amount of said VEGF siRNA can be administered every eight weeks. The effective amount of said VEGF siRNA can be administered every twelve weeks.
The present invention further provides a method of inhibiting choroidal neovascularization in a subject comprising administering to the subject an effective amount of an siRNA comprising a sense RNA strand of SEQ ID NO: 77 and an antisense RNA strand of SEQ ID NO: 78.
The present invention further provides a method of treating diabetic macular edema in a subject comprising administering to the subject an effective amount of an siRNA comprising a sense RNA strand of SEQ ID NO: 77 and an antisense RNA strand of SEQ ID NO: 78.
The sense and antisense RNA strands can be stabilized against nuclease degradation.
The siRNA can be administered by an intraocular administration route, optionally the intraocular administration route is selected from intravitreal, intraretinal, subretinal, subtenon, peri- and retro-orbital, trans-corneal and trans-scleral administration.
The above method of the invention can further comprise administering a VEGF antagonist. Optionally the VEGF antagonist is a monoclonal antibody targeting human VEGF selected from bevacizumab and ranibizumab, preferably the VEGF antagonist is ranibizumab.
In the above method of the invention, the effective amount can be from about 0.5 mg to about 5 mg. The effective amount can be about 2.5 mg.
The effective amount of said VEGF siRNA can be administered every four weeks. The effective amount of said VEGF siRNA can be administered every eight weeks. The effective amount of said VEGF siRNA can be administered every twelve weeks.
The present invention further provides a method of decreasing foveal thickness in a subject comprising administering to the subject an effective amount of an siRNA comprising a sense RNA strand of SEQ ID NO: 77 and an antisense RNA strand of SEQ ID NO: 78.
The sense and antisense RNA strands can be stabilized against nuclease degradation.
The siRNA can be administered by an intraocular administration route, optionally the intraocular administration route is selected from intravitreal, intraretinal, subretinal, subtenon, peri- and retro-orbital, trans-corneal and trans-scleral administration.
The above method of the invention can further comprise administering a VEGF antagonist. Optionally the VEGF antagonist is a monoclonal antibody targeting human VEGF selected from bevacizumab and ranibizumab, preferably the VEGF antagonist is ranibizumab.
In the above method of the invention, the effective amount can be from about 0.5 mg to about 5 mg. The effective amount can be about 2.5 mg.
The effective amount of said VEGF siRNA can be administered every four weeks. The effective amount of said VEGF siRNA can be administered every eight weeks. The effective amount of said VEGF siRNA can be administered every twelve weeks.
The present invention additionally provides a method of treating age-related macular degeneration comprising administering to a subject an effective amount of a VEGF antagonist and an effective amount of an siRNA comprising a sense RNA strand and an antisense RNA strand, wherein the sense and the antisense RNA strands form an RNA duplex, and wherein the sense RNA strand comprises a nucleotide sequence identical to a target sequence of about 19 to about 25 contiguous nucleotides in human VEGF mRNA.
The sense RNA strand can comprise SEQ ID NO: 77 and the antisense strand can comprise SEQ ID NO: 78.
The siRNA can be administered by an intraocular administration route, optionally the intraocular administration route is selected from intravitreal, intraretinal, subretinal, subtenon, peri- and retro-orbital, trans-corneal and trans-scleral administration.
The effective amount of said siRNA can be from about 0.5 mg to about 5 mg. The effective amount of said siRNA can be about 2.5 mg.
In the above method of the invention the VEGF antagonist can be administered prior to administration of the siRNA.
Alternatively, in the above method of the invention the VEGF antagonist can be administered after administration of the siRNA.
Alternatively, in the above method of the invention the VEGF antagonist can be administered simultaneously with administration of the siRNA.
The effective amount of said VEGF siRNA can be administered every four weeks. The effective amount of said VEGF siRNA can be administered every eight weeks. The effective amount of said VEGF siRNA can be administered every twelve weeks.
The VEGF antagonist can be ranibizumab. In one embodiment the ranibizumab can be administered two weeks prior to administration of said siRNA, and in this embodiment said siRNA can be administered every four weeks and said ranibizumab is administered every four weeks on an alternating basis. Thus for example, the ranibizumab can be administered over an eight week period, and optionally said siRNA can be administered on a maintenance basis after the eight week period.
The VEGF antagonist can be bevacizumab.
The VEGF antagonist can be aflibercept.
The VEGF antagonist can be pegaptanib.
The present invention additionally provides a method of treating diabetic macular edema comprising administering to a subject an effective amount of a VEGF antagonist and an effective amount of an siRNA comprising a sense RNA strand and an antisense RNA strand, wherein the sense and the antisense RNA strands form an RNA duplex, and wherein the sense RNA strand comprises a nucleotide sequence identical to a target sequence of about 19 to about 25 contiguous nucleotides in human VEGF mRNA.
The siRNA can be administered by an intraocular administration route, optionally the intraocular administration route is selected from intravitreal, intraretinal, subretinal, subtenon, peri- and retro-orbital, trans-corneal and trans-scleral administration.
The effective amount of said siRNA can be from about 0.1 mg to about 5 mg. The effective amount of said siRNA can be about 2.5 mg.
The VEGF antagonist can be a monoclonal antibody targeting human VEGF selected from bevacizumab and ranibizumab, preferably the VEGF antagonist is ranibizumab. In the above method of the invention the VEGF antagonist can be administered prior to administration of the siRNA.
Alternatively, in the above method of the invention the VEGF antagonist can be administered after administration of the siRNA.
Alternatively, in the above method of the invention the VEGF antagonist can be administered simultaneously with administration of the siRNA.
The effective amount of said VEGF siRNA is administered every four weeks. The effective amount of said VEGF siRNA can be administered every eight weeks. The effective amount of said VEGF siRNA can be administered every twelve weeks.

## Claims

1. An siRNA comprising a sense RNA strand of SEQ ID NO: 77 and an antisense strand of SEQ ID NO: 78 for use in treating choroidal revascularization in a human subject.

2. The siRNA of claim 1, wherein the sense and antisense RNA strands are stabilized against nuclease degradation.

3. The siRNA of claim 2, wherein the siRNA is suitable for administration by an intraocular administration route, preferably wherein the intraocular administration route is selected from intravitreal, intraretinal, subretinal, subtenon, peri- and retro-orbital, trans-corneal and trans-scleral administration.

4. The siRNA of any one of claims 1 to 3 wherein a VEGF antagonist is also administered.

5. The siRNA of claim 4, wherein the VEGF antagonist is a monoclonal antibody targeting human VEGF selected from bevacizumab and ranibizumab, preferably wherein the VEGF antagonist is ranibizumab.

6. The siRNA of any one of claims 1 to 5, wherein the siRNA is administered in an effective amount from about 0.5 mg to about 5 mg, preferably wherein the effective amount is about 2.5 mg.

7. The siRNA of any one of claims 1 to 6, wherein the siRNA is for administration once every four weeks.

8. The siRNA of any one of claims 1 to 6, wherein the siRNA is for administration once every eight weeks.

9. The siRNA of any one of claims 1 to 6, wherein the siRNA is for administration once every twelve weeks.

10. Use of an siRNA comprising a sense RNA strand of SEQ ID NO: 77 and an antisense RNA strand of SEQ ID NO: 78 in the manufacture of a medicament for treating choroidal revascularization in a human subject.

11. A combination of an effective amount of (i) a VEGF antagonist and (ii) an siRNA comprising a sense RNA strand and an antisense RNA strand, wherein the sense and the antisense RNA strands form an RNA dulex, and wherein the sense RNA strand comprises a nucleotide sequence identical to a target sequence of about 19 to about 25 contiguous nucleotides in human VEGF mRNA for use in treating choroidal revascularization in a human subj ect.

12. The combination as claimed in claim 11 wherein said VEGF antagonist is for administration prior to or after administration of the siRNA.

13. The combination as claimed in claim 11 wherein said VEGF antagonist is for administration simultaneously with said siRNA.

14. The combination of any one of claims 11 to 13, wherein the siRNA is suitable for administration by an intraocular administration route, preferably wherein the intraocular administration route is selected from intravitreal, intraretinal, subretinal, subtenon, peri- and retro-orbital, trans-corneal and trans-scleral administration.

15. The combination of any one of claims 11 to 14, wherein the siRNA is administered in an effective amount from about 0.5 mg to about 5 mg, preferably wherein the effective amount is about 2.5 mg.

16. The combination of any one of claims 11 to 15, wherein the VEGF antagonist is a monoclonal antibody targeting human VEGF selected from bevacizumab and ranibizumab, preferably wherein the VEGF antagonist is ranibizumab.

17. The combination of any one of claims 11 to 16, wherein the siRNA is for administration once every four weeks.

18. The combination of any one of claims 11 to 16, wherein the siRNA is for administration once every eight weeks.

19. The combination of any one of claims 11 to 16, wherein the siRNA is for administration once every twelve weeks.
